(19) Europäisches Patentamt
European Patent Office
Office européen des brevets

(11) EP 4 374 698 B1

(12) **EUROPEAN PATENT SPECIFICATION**

(45) Date of publication and mention of the grant of the patent:
**20.08.2025 Bulletin 2025/34**

(21) Application number: **22209905.3**

(22) Date of filing: **28.11.2022**

(51) International Patent Classification (IPC):
***A01N 43/40*** *(2006.01)* ***A01P 1/00*** *(2006.01)*
***C07D 213/79*** *(2006.01)* ***C07D 213/80*** *(2006.01)*

(52) Cooperative Patent Classification (CPC):
**A01N 43/40; A01P 1/00; C07D 213/55**

(54) **PYRIDINIUM ESTERS**

PYRIDINIUMESTER

ESTERS DE PYRIDINIUM

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**29.05.2024 Bulletin 2024/22**

(73) Proprietor: **The Procter & Gamble Company**
**Cincinnati, OH 45202 (US)**

(72) Inventors:
- **MASH, Brandon L.**
  **Cincinnati, 45202 (US)**
- **NATOLI, Sean Nicholas**
  **Cincinnati, 45202 (US)**
- **MIRACLE, Gregory Scot**
  **Cincinnati, 45202 (US)**

(74) Representative: **P&G Patent Belgium UK**
**Temselaan 100**
**1853 Strombeek-Bever (BE)**

(56) References cited:
**WO-A1-2021/022286 CN-A- 102 796 502**

- **LUKES R ET AL: "Reduktion von Pyridinbasen durch Ameisensäure XI. Reduktion von 3-(2-Pyridyl)acrylsäure- und 3-(4-Pyridyl)acrylsäure-methylbetain [Reduction of pyridine bases with formic acid. XI. Reduction of 3-(2-pyridyl)acrylic and 3-(4-pyridyl)acrylic acid methyl betaines]", COLLECTION OF CZECHOSLOVAK CHEMICAL COMMUNICATIONS,, vol. 25, no. 10, 1 October 1960 (1960-10-01), pages 2668 - 2674, XP009543730, ISSN: 0010-0765, DOI: 10.1135/CCCC19602668**
- **LIVSHITS R S ET AL: "Isoquinoline compounds. X. Synthesis of 1-[2-(-decyl-3-piperidyl) ethyl]-6,7-dimethoxy-1,2,3,4-tetrahydroisoquinoline", JOURNAL OF GENERAL CHEMISTRY USSR, CONSULTANTS BUREAU, NEW YORK, NY, US, vol. 23, 1 January 1953 (1953-01-01), pages 541 - 543, XP009543744, ISSN: 0022-1279**
- **MIRONOV ANDREJ ET AL: "Study in the pyridine series. VII. Infrared spectra of some [delta]3-piperideines", SBORNIK VYSOKE SKOLY CHEMICKO-TECHNOLOGICKE V PRAZE, ORGANICKA TECHNOLOGIE,, vol. 5, 1 January 1961 (1961-01-01), pages 83 - 91, XP009543729**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

**Description**

FIELD OF THE INVENTION

The present invention relates to pyridinium esters and compositions comprising the esters.

BACKGROUND OF THE INVENTION

Antibacterial household compositions are becoming more widely used with a focus on kitchen, bath and toilet cleaning. In addition to the sanitization or disinfection the compositions are required to clean and/or provide shine to the treated surface. Although there exists a number of antimicrobial treatment compositions, there is a desire to find more efficient and more environmentally friendly compositions. Furthermore, the development of isotropic formulations with gentle, biodegradable actives is desirable.

There is therefore the need for an antibacterial treatment composition that has a good environmental and human safety profile, that shows strong antibacterial efficacy, that can be used on food contact surfaces and that shows good cleaning, even on tough greasy soils, and/or leaves the surfaces shiny and without streaks. WO2021022286A1 describes an antimicrobial cleaning composition comprising a bispyridinium alkane antimicrobial active and being substantially free of a quaternary ammonium antimicrobial active.

SUMMARY OF THE INVENTION

According to the first aspect of the present invention, there is provided a pyridinium ester as defined in claim 1.

According to the second aspect of the invention, as defined in claim 4, there is provided an antimicrobial composition comprising an antimicrobial system, the antimicrobial system comprising a surfactant, a solvent, and the pyridinium ester of the invention.

According to the third aspect of the present invention, as defined in claim 14, there is provided an article treated with the composition of the second aspect of the invention. The article is in the form of a disposable or partially reusable substrate comprising one or more nonwoven layers. The article provides sanitization to surfaces, in particular hard surfaces. The article is sometimes herein referred to as "the article of the invention".

According to the fourth aspect of the present invention, as defined in claim 15, there is provided a method of sanitizing an inanimate surface comprising the step of contacting the surface with the composition or article of the invention. There is also provided the use of the composition of the invention to provide antimicrobial benefits to an inanimate surface, as defined in claim 16.

The elements of the of the invention described in relation to any of the aspects of the invention apply *mutatis mutandis* to the other aspects of the invention.

DETAILED DESCRIPTION OF THE INVENTION

The present invention relates to pyridinium esters and compositions comprising the pyridinium esters. The composition provides antimicrobial benefits. The composition comprises an antimicrobial system. The system comprises three components that combined provide a very effective antimicrobial action. The composition is preferably an aqueous composition comprising at least 50% by weight of the composition of water. The composition is especially suitable for treating hard surfaces.

As used herein, the phrase "chain atoms" means the sum of all atoms in an indicated group or moiety, excluding hydrogen atoms. The chain atoms may be in a linear constitution, a branched constitution, and/or a ring constitution.

As used herein, "hydrocarbyl group" means any univalent radical, derived from a hydrocarbon.

All percentages, ratios and proportions used herein are by weight percent of the composition, unless otherwise specified. All average values are calculated "by weight" of the composition, unless otherwise expressly indicated. All ratios are calculated as a weight/weight level, unless otherwise specified.

All measurements are performed at 25°C unless otherwise specified.

Unless otherwise noted, all component or composition levels are in reference to the active portion of that component or composition, and are exclusive of impurities, for example, residual solvents or by-products, which may be present in commercially available sources of such components or compositions.

Pyridinium ester

The pyridinium ester of the invention has the following formula (Formula I)

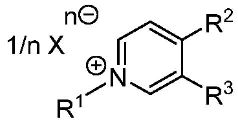 Formula I,

wherein 1/n $X^{n-}$ is a counterion;

wherein n is 1 or 2;

wherein one of $R^2$ and $R^3$ is H and the other is selected from the group consisting of Formula II, Formula III and Formula IV ,

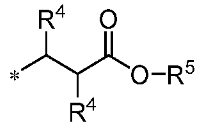

Formula II

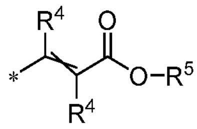

Formula III

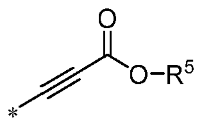

Formula IV

wherein * represents the point of attachment to the heteroaryl ring,

Formula III can have an *E*- or *Z*- configuration, Formula III can be selected from the group consisting of a *Z*-configuration, an E-configuration, and mixtures thereof, preferably an E-configuration,

wherein when $R^2$ is H, $R^1$ is a branched or unbranched, saturated or unsaturated, $C_4$-$C_{12}$ hydrocarbyl group, preferably $C_4$-$C_{10}$, more preferably $C_4$-$C_8$ and especially $C_6$ hydrocarbyl group;

when $R^3$ is H, $R^1$ is a branched or unbranched, saturated or unsaturated, $C_1$-$C_{12}$ hydrocarbyl group, preferably $C_2$-$C_{10}$, more preferably $C_4$-$C_8$ and especially $C_6$ hydrocarbyl group;
and

wherein each $R^4$ is independently selected from hydrogen and a $C_1$-$C_4$ hydrocarbyl group and

wherein $R^5$ is a branched or unbranched, saturated or unsaturated, $C_4$-$C_{12}$ hydrocarbyl group, preferably $R^5$ is a branched or unbranched $C_6$-$C_8$ saturated hydrocarbyl group, more preferably a $C_6$ hydrocarbyl group.

Pyridinium esters wherein one of $R_2$ and $R_3$ is H and the other is Formula III, provide good antimicrobial properties when used as part of the antimicrobial system comprising a surfactant and a solvent. Preferred pyridinium esters are those in which one of $R_2$ and $R_3$ is H and the other is Formula III, wherein all $R_4$ are H, and wherein $R_1$ and $R_5$ are independently selected from a branched or unbranched $C_6$-$C_8$ saturated hydrocarbyl groups, preferably $R_1$ and $R_5$ are independently selected from unbranched $C_6$ saturated hydrocarbyl groups.

Preferably, Formula III is

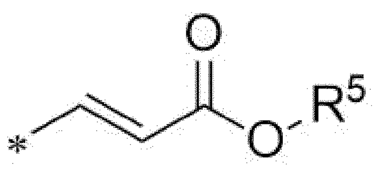

More preferably R5 is a $C_6$-$C_8$ hydrocarbyl group, especially $C_6H_{13}$.

Preferably, the pyridinium ester is selected from the group consisting of:

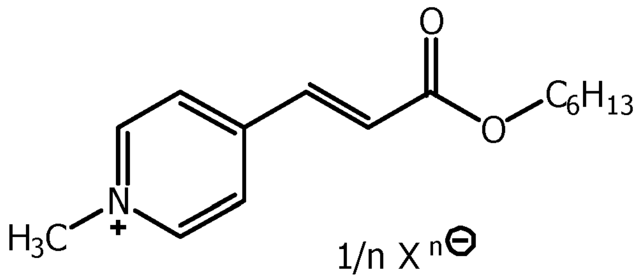

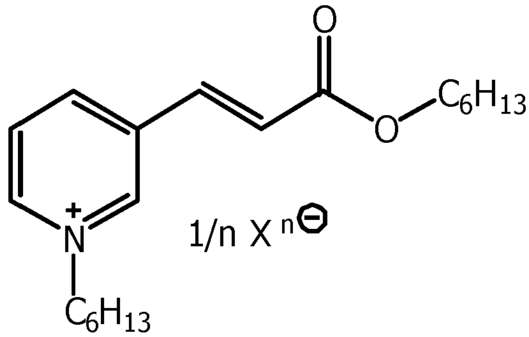

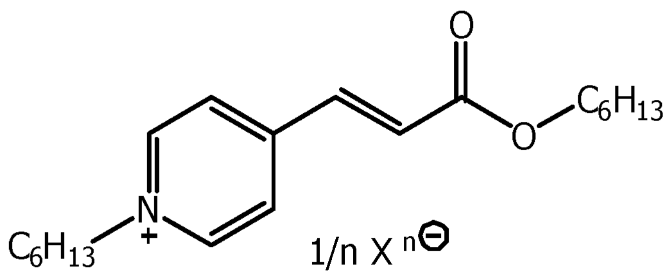

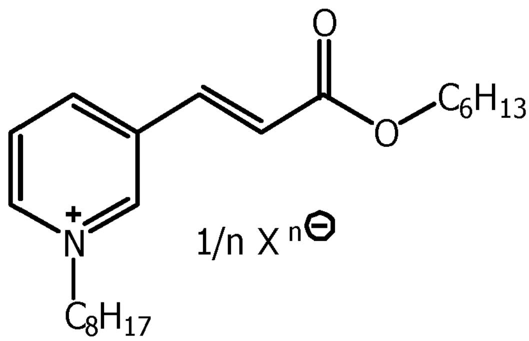

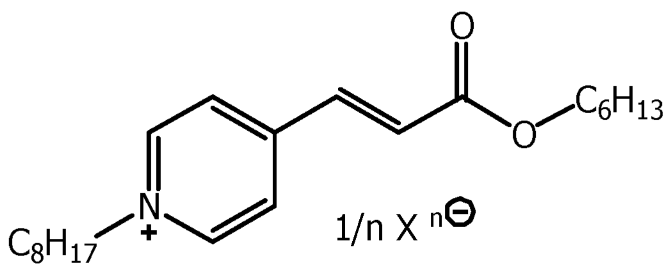

Especially preferred are the pyridinium esters are selected from the group consisting of:

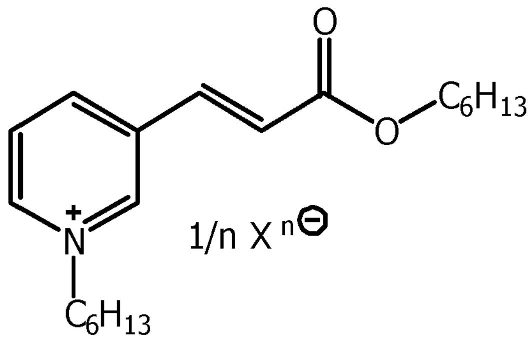

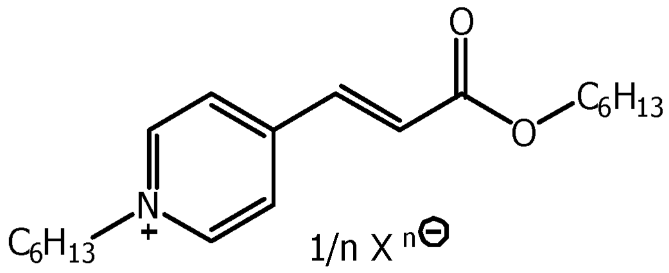

and mixtures thereof.

Mixtures of the pyridinium esters of the invention can also be good in terms of antimicrobial properties when part of the antimicrobial system comprised in the antimicrobial composition of the invention.

The pyridinium esters of the invention can be obtained by reacting pyridine carboxaldehydes with malonic acid mono-esters in the presence of pyridine and amino acids such as alanine as catalysts. The resulting compounds are modified by adding hydrocarbon moieties both to the pyridine nitrogen atom and to the terminal carboxylate oxygen atom. Hydrocarbon moieties are selected to increase the overall hydrophobicity of the resulting compounds, since this is believed to improve antimicrobial efficacy.

An example is illustrated herein below:

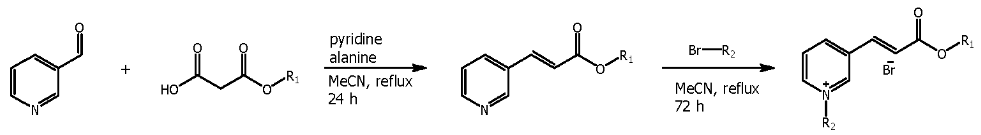


Examples of starting materials suitable for preparing the pyridinium esters of the invention, in particular alkyl pyridinium esters include, but are not limited to, Ethyl (2E)-3-(3-pyridinyl)-2-propenoate (prepared as described in: Synlett (2011), (12), 1723-1726); 4-Pyridinepropanoic acid, ethyl ester (Prepared as described in: Journal of the American Chemical Society (2011), 133(3), 582-594); Ethyl (2E)-3-(4-pyridinyl)-2-propenoate (synthesized according to the procedure described in: Organic Letters (1999), 1(4), 579-582); Ethyl 3-pyridinepropanoate (Prepared as disclosed in: Bioorganic & Medicinal Chemistry (2009), 17(5), 2047-2068); Ethyl 3-(3-pyridinyl)-2-propynoate (prepared from the acid, the preparation of which is given in the Journal of Heterocyclic Chemistry (1981), 18(3), 519-23, according to: Zhurnal Organicheskoi Khimii (1976), 12(2), 443-8); Ethyl (2Z)-3-(3-pyridinyl)-2-propenoate (prepared as described in: Tetrahedron Letters (2018), 59(1), 14-17); and Ethyl 3-(4-pyridinyl)-2-propynoate (prepared as described in: WO2002/060438 A1).

Examples of starting materials suitable for preparing the pyridinium esters of the invention, in particular α-alkylated alkyl pyridinium esters include, but are not limited to, Ethyl 2-(3-pyridinylmethylene)butanoate, Ethyl 2-(4-pyridinylmethylene)butanoate, Ethyl α-(1-methylethyl)-4-pyridinepropanoate, Ethyl α-(1-methylethyl)-3-pyridinepropanoate, Ethyl α-ethyl-4-pyridinepropanoate, and Ethyl α-ethyl-3-pyridinepropanoate (available for purchase from Aurora Fine Chemicals, LLC. San Diego, CA, USA); Methyl (2Z)-2-methyl-3-(4-pyridinyl)-2-propenoate and 2-Propenoic acid, 2-methyl-3-(3-pyridinyl)-, ethyl ester, (2Z)- (prepared as disclosed in: Chemistry - A European Journal (2013), 19(45), 15281-15289); Ethyl (2E)-2-methyl-3-(3-pyridinyl)-2-propenoate (prepared as disclosed in: Chemistry - A European Journal (2009), 15(18), 4538-4542), which upon hydrogenation yields Ethyl α-methyl-4-pyridinepropanoate; and Ethyl (2E)-2-methyl-3-(4-pyridinyl)-2-propenoate (prepared as disclosed in: ChemPlusChem (2016), 81(8), 893-898), which upon hydrogenation yields Ethyl α-methyl-4-pyridinepropanoate.

Examples of starting materials suitable for preparing the pyridinium esters of the invention, in particular β-alkylated alkyl pyridinium esters include, but are not limited to, Ethyl (2E)-3-(3-pyridinyl)-2-butenoate, Ethyl β-methyl-3-pyridinepropanoate, Ethyl (2Z)-3-(3-pyridinyl)-2-butenoate, Ethyl (2Z)-3-(4-pyridinyl)-2-butenoate, Ethyl (2E)-3-(4-pyridinyl)-2-butenoate, Methyl β-ethyl-3-pyridinepropanoate, Methyl β-ethyl-4-pyridinepropanoate, Methyl β-propyl-3-pyridinepropanoate, Methyl 3-(4-pyridinyl)-2-butenoate, Ethyl β-ethyl-4-pyridinepropanoate, Ethyl β-ethyl-3-pyridinepropanoate, Ethyl p-propyl-3-pyridinepropanoate, Ethyl β-(1-methylpropyl)-4-pyridinepropanoate, and Ethyl β-(1-methylpropyl)-3-pyridinepropanoate (all available for purchase from Aurora Fine Chemicals, LLC. San Diego, CA, USA); Ethyl β-methyl-4-pyridinepropanoate (available for purchase from Enamine US Inc., Monmouth Junction, NJ, USA); Ethyl 3-(4-pyridinyl)-2-butenoate (prepared as disclosed in WO 2001/085714 A1); 3-Pyridinepropanoic acid, β-propyl-, ethyl ester, (βS)-; 4-Pyridinepropanoic acid, β-propyl-, ethyl ester, (βS)-; 3-Pyridinepropanoic acid, β-2-methylpropyl-, ethyl ester, (βS)-; and 4-Pyridinepropanoic acid, β-2-methylpropyl-, ethyl ester, (βS)- (all prepared as disclosed in: ACS Catalysis (2022), 12(4), 2434-2440); and Methyl (2E)-3-(3-pyridinyl)-2-hexenoate and Methyl (2Z)-3-(3-pyridinyl)-2-hexenoate (prepared as disclosed in: Chemical Communications (Cambridge, United Kingdom) (2020), 56(7), 1101-1104).

Examples of starting materials suitable for preparing the pyridinium esters of the invention, in particular α,β-alkylated alkyl pyridinium esters include, but are not limited to, Ethyl (2Z)-2-methyl-3-(3-pyridinyl)-2-butenoate; 2-Butenoic acid, 2-methyl-3-(3-pyridinyl)-, ethyl ester; Ethyl 2-methyl-3-(4-pyridinyl)-2-butenoate; Ethyl 2-ethyl-3-(3-pyridinyl)-2-butenoate; and Ethyl 2-ethyl-3-(4-pyridinyl)-2-butenoate (all available for purchase from Aurora Fine Chemicals, LLC. San Diego, CA, USA); and Ethyl (2E)-2-methyl-3-(3-pyridinyl)-2-butenoate (available for purchase from Enamine US Inc., Monmouth Junction, NJ, USA).

Any of the starting esters listed above can be converted to an ester of an alcohol $R^5$-OH via a simple transesterification reaction, wherein $R^5$ is a branched or unbranched, saturated or unsaturated, $C_4$-$C_{12}$ hydrocarbyl group. The pyridinium esters of the invention may then be prepared through quaternization of the pyridine nitrogen via typical nucleophilic displacement of a suitable leaving group X from a compound such as $R^1$-X, wherein $R^1$ is a branched or unbranched, saturated or unsaturated, $C_1$-$C_{12}$ or $C_4$-$C_{12}$ hydrocarbyl group.

Examples of suitable counterion X (charge balancing anions to form the salts) include but are not limited to: fluoride, chloride, bromide, iodide, perchlorate, hydrogen sulfate, sulfate, aminosulfate, nitrate, dihydrogen phosphate, hydrogen phosphate, phosphate, bicarbonate, carbonate, methosulfate, ethosulfate, cyanate, thiocyanate, tetrachlorozincate, borate, tetrafluoroborate, acetate, chloroacetate, cyanoacetate, hydroxyacetate, aminoacetate, methylaminoacetate, di- and tri-chloroacetate, 2-chloro-propionate, 2-hydroxypropionate, glycolate, thioglycolate, thioacetate, phenoxyacetate, trimethylacetate, valerate, palmitate, acrylate, oxalate, malonate, crotonate, succinate, citrate, methylene-bis-thioglycolate, ethylene-bis-iminoacetate, nitrilotriacetate, fumarate, maleate, benzoate, methylbenzoate, chlorobenzoate, dichlorobenzoate, hydroxybenzoate, aminobenzoate, phthalate, terephthalate, indolylacetate, chlorobenzenesulfonate, benzenesulfonate, toluenesulfonate, biphenyl-sulfonate and chlorotoluenesulfonate. Those of ordinary skill in the art are well aware of different counterions which can be used in place of those listed above. Preferably, the charge balancing anion have a molecular weight below 200, counterions suitable for the pyridinium esters of the invention are listed in the table herein below. Specially preferred for use herein are halides, in particular chloride and bromide.

| Anion Name | Empirical Formula | MW |
|---|---|---|
| Methanesulfonate | $CH_3SO_3$ | 95.1 |
| Ethanesulfonate | $CH_3CH_2SO_3$ | 109.12 |
| Benezenesulfonate | $C_6H_5SO_3$ | 157.17 |
| p-Toluenesulfonate | $CH_3C_6H_4SO_3$ | 171.19 |
| Cumenesulfonate | $C_9H_{11}O_3S$ | 199.25 |
| Xylenesulfonate | $(CH_3)_2C_6H_3SO_3$ | 185.22 |
| Chloride | Cl | 35.45 |
| Bromide | Br | 79.90 |

The pyridinium esters of the invention are very resilient in terms of pH, i.e., they maintain their antimicrobial properties across a broad pH range. The pyridinium esters of the invention present a good environmental profile.

Antimicrobial treatment composition

The composition of the invention is suitable to be used on surfaces, preferably on inanimate surfaces, more preferably on hard surfaces. The composition can be delivered onto the surface, by for example spraying the composition, followed by wiping the surface, preferably without rinsing or by using a substrate, such as a wipe impregnated with the

composition of the invention. The composition provides good cleaning and/or shine to the treated surface. The composition can be a concentrated composition, that can be diluted before use or a ready to use composition. Preferably, the composition is a ready-to-use sprayable composition.

As used herein, the terms "microbe" or "microbial" should be interpreted to refer to any of the microscopic organisms studied by microbiologists or found in the use environment of a treated surface. Such organisms include, but are not limited to, bacteria and fungi as well as other single-celled organisms such as mould, mildew and algae. Viruses (enveloped and non-enveloped) and other infectious agents are also included in the term microbe.

"Antimicrobial" further should be understood to encompass both microbiocidal and microbiostatic properties. That is, the term includes microbe killing, leading to a reduction in number of microbes, as well as a retarding effect of microbial growth, wherein numbers may remain more or less constant (but nonetheless allowing for slight increase/decrease).

For ease of discussion, this description uses the term antimicrobial to denote a broad-spectrum activity (e.g. against bacteria and fungi, or against bacteria and viruses). When speaking of efficacy against a particular microorganism or taxonomic rank, the more focused term will be used (e.g. antifungal to denote efficacy against fungal growth in particular). Using the above example, it should be understood that efficacy against fungi does not in any way preclude the possibility that the same antimicrobial composition may demonstrate efficacy against another class of microbes.

By "hard surface ", it is meant herein hard surfaces found in households, especially domestic households. Surfaces to be cleaned include kitchens and bathrooms, e.g., floors, walls, tiles, windows, cupboards, sinks, showers, shower plastified curtains, wash basins, WCs, fixtures and fittings and the like made of different materials like ceramic, vinyl, no-wax vinyl, linoleum, melamine, glass, steel, kitchen work surfaces, any plastics, plastified wood, metal or any painted or varnished or sealed surface and the like. Household hard surfaces also include household appliances including, but not limited to refrigerators, freezers, washing machines, automatic dryers, ovens, microwave ovens, dishwashers and so on. Such hard surfaces may be found both in private households as well as in commercial, institutional and industrial environments.

The compositions herein are aqueous compositions, comprising at least 50% by weight of the composition of water, preferably from 80% to 95% and more preferably from 90% to 96% by weight of the composition of water. Preferably, the compositions of the invention are clear compositions. Without wishing to be bound by theory, it is believed that in order to provide the best antimicrobial efficacy, the antimicrobial system should be in solution. The composition should preferably be in the form of a clear, isotropic liquid.

The compositions of the present invention preferably can be non-thickened, or water like, having a viscosity of from 1 mPa.s to 5 Pa.s, or can be thickened, having a viscosity of from 50 Pa.s to 1200 Pa.s, more preferably 100 Pa.s to 800 Pa.s, most preferably 200 Pa.s to 600 Pa.s when measured at 20°C with a AD1000 Advanced Rheometer from Atlas® shear rate 10 $s^{-1}$ with a coned spindle of 40mm with a cone angle 2° and a truncation of $\pm 60 \mu m$.

## Antimicrobial system

The composition of the invention comprises an antimicrobial system comprising a pyridinium ester. The pyridinium ester needs only be present in germicidal effective amounts, which can be as little as 0.001% to less than 10% by weight of the composition. In more preferred compositions, the cleaning composition comprises the pyridinium ester, or a mixture thereof, at a level of from from about 0.0025 to about 5%, preferably from 0.005% to 1.5% by weight of the composition.

A germicidal effective amount of the antimicrobial agent typically results in at least a log 4 reduction of gram-negative bacteria, using the method of EN13697 (Chemical Disinfectants Bactericidal Activity Testing), in 5 minutes.

## Surfactant

The composition of the invention preferably comprises surfactants, more preferably from 0.01% to 10%, preferably from 0.05% to 8%, more preferably from 0.08% to 5%, more preferably 0.1% to 3% by weight of the composition of surfactant. The surfactant also contributes to the cleaning and/or shine provided by the composition.

## Alkoxylated alcohol nonionic surfactants

Suitable alkoxylated alcohol nonionic surfactants are according to the formula $RO-(A)_nH$, wherein: R is a primary $C_4$ to $C_{18}$, preferably a $C_6$ to $C_{16}$, more preferably a $C_{10}$ to $C_{16}$ and even more preferably from $C_{12}$ to $C_{14}$ branched or linear alkyl chain, or a $C_6$ to $C_{28}$ alkyl benzene chain; A is an ethoxy or propoxy or butoxy unit, or mixtures thereof, and wherein n is an integer from 1 to 30, preferably from 1 to 15, more preferably from 3 to 12 even more preferably from 3 to 8. Preferred R chains for use herein are the $C_{10}$ to $C_{16}$ linear or branched alkyl chains. Especially preferred for use herein is an alcohol alkoxylated having a $C_{12}$-$C_{14}$ chain length and having from 8 to 10 ethoxy groups.

Suitable branched alkoxylated alcohols may be selected from the group consisting of: $C_4$-$C_{10}$ alkyl branched alkoxylated alcohols, and mixtures thereof. The branched alkoxylated alcohol can be derived from the alkoxylation of $C_4$-$C_{10}$ alkyl branched alcohols selected form the group consisting of: $C_4$-$C_{10}$ primary mono-alcohols having one or more $C_1$-$C_4$ branching groups.

By $C_4$-$C_{10}$ primary mono-alcohol, it is meant that the main chain of the primary mono-alcohol has a total of from 4 to 10 carbon atoms. The $C_4$-$C_{10}$ primary mono-alcohol can be selected from the group consisting of: methyl butanol, ethyl butanol, methyl pentanol, ethyl pentanol, methyl hexanol, ethyl hexanol, propyl hexanol, dimethyl hexanol, trimethyl hexanol, methyl heptanol, ethyl heptanol, propyl heptanol, dimethyl heptanol, trimethyl heptanol, methyl octanol, ethyl octanol, propyl octanol, butyl octanol, dimethyl octanol, trimethyl octanol, methyl nonanol, ethyl nonanol, propyl nonanol, butyl nonanol, dimethyl nonanol, trimethyl nonanol and mixtures thereof.

The $C_4$-$C_{10}$ primary mono-alcohol can be selected from the group consisting of: ethyl hexanol, propyl hexanol, ethyl heptanol, propyl heptanol, ethyl octanol, propyl octanol, butyl octanol, ethyl nonanol, propyl nonanol, butyl nonanol, and mixtures thereof. Preferably the $C_4$-$C_{10}$ primary mono-alcohol is selected from the group consisting of: ethyl hexanol, propyl hexanol, ethyl heptanol, propyl heptanol, and mixtures thereof.

The $C_4$-$C_{10}$ primary mono-alcohol is most preferably ethyl hexanol, and propyl heptanol. Especially preferred for use herein are ethoxylated ethyl hexanol comprising from 4 to 10 ethoxy groups.

In the branched alkoxylated alcohol, the one or more $C_1$-$C_4$ branching group can be substituted into the $C_4$-$C_{10}$ primary mono-alcohol at a C1 to C3 position, preferably at the C1 to C2 position, more preferably at the C2 position, as measured from the hydroxyl group of the starting alcohol.

The branched alkoxylated alcohol can comprise from 1 to 14, preferably from 2 to 7, more preferably from 4 to 6 ethoxylate units, and optionally from 1 to 9, preferably from 2 to 7, more preferably from 4 to 6 of propoxylate units.

The branched alkoxylated alcohol is preferably 2-ethyl hexan-1-ol ethoxylated to a degree of from 4 to 6, and propoxylated to a degree of from 4 to 6, more preferably, the alcohol is first propoxylated and then ethoxylated. Another preferred branched alkoxylated alcohols are 2-alkyl-1-alkanols such as alkoxylated $C_{10}$ guerbet alcohols with 1 to 14, preferably from 2 to 7, more preferably from 3 to 6 ethoxylate or ethoxylate-propoxylate units.

Non-limiting examples of suitable branched alkoxylated alcohols are, for instance, Ecosurf® EH3, EH6, and EH9, commercially available from DOW, and Lutensol® XP alkoxylated Guerbet alcohols & Lutensol® XL ethoxylated Guerbet alcohols available from BASF.

Linear alcohol alkoxylated nonionic surfactants preferred herein are alkoxylated nonionic surfactants with a $C_8$, $C_{10}$, $C_{12}$, mixtures of $C_8$ to $C_{10}$, mixtures of $C_{10}$ to $C_{12}$, mixtures of $C_9$ to $C_{11}$ linear alkyl chain and 8 or less ethoxylate units, preferably 3 to 8 ethoxylate units.

Non-limiting examples of suitable linear alkoxylated nonionic surfactants for use herein are Dobanol® 91-2.5 (R is a mixture of $C_9$ and $C_{11}$ alkyl chains, n is 2.5), Dobanol® 91-5 (R is a mixture of $C_9$ to $C_{11}$ alkyl chains, n is 5); Dobanol® 91-10 (R is a mixture of $C_9$ to $C_{11}$ alkyl chains, n is 10); Greenbentine DE60 (R is a C10 linear alkyl chain, n is 6); Marlipal 10-8 (R is a $C_{10}$ linear alkyl chain, n is 8); Neodol 91-8 (R is a mixture of $C_9$ to $C_{11}$ alkyl chains, n is 8); Empilan® KBE21 (R is a mixture of $C_{12}$ and $C_{14}$ alkyl chains, n is 21); Lutensol ON30 (R is $C_{10}$ linear alkyl chain, n is 3); Lutensol ON50 (R is $C_{10}$ linear alkyl chain, n is 5); Lutensol ON70 (R is $C_{10}$ linear alkyl chain, n is 7); Novel 610-3.5 (R is mixture of $C_6$ to $C_{10}$ linear alkyl chains, n is 3.5); Novel 810FD-5 (R is mixture of $C_8$ to $C_{10}$ linear alkyl chains, n is 5); Novel 10-4 (R is $C_{10}$ linear alkyl chain, n is 4); Novel 1412-3 (R is mixture of $C_{12}$ to $C_{14}$ linear alkyl chains, n is 3); Lialethl® 11-5 (R is a $C_{11}$ linear alkyl chain, n is 5); Lialethl® 11-21 (R is a mixture of linear and branched $C_{11}$ alkyl chain, n is 21), or mixtures thereof.

The alkoxylated nonionic surfactant may be a secondary alcohol ethoxylate such as for example the Tergitol™-15-S surfactants having the general formula shown below and commercially available from DOW

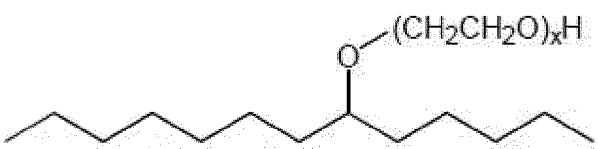

Alkyl carbon chain length = 11 to 15, X = 3 to 40

Tergitol 15-S surfactants

Preferred secondary alcohol ethoxylate surfactants have 3-9 EO units.

Another suitable alkoxylated nonionic surfactant is an alkyl ethoxy alkoxy alcohol, preferably wherein the alkoxy part of the molecule is propoxy, or butoxy, or propoxy-butoxy. More preferred alkyl ethoxy alkoxy alcohols are of formula (II):

$$RO\text{-}(CH_2CH_2O)_m(CH_2\overset{R^1}{\overset{|}{C}}HO)_n\text{ - }H \qquad \text{Formula (II)}$$

wherein:

R is a branched or unbranched alkyl group having 8 to 16 carbon atoms;
$R^1$ is a branched or unbranched alkyl group having 1 to 5 carbon atoms;
n is an integer from 1 to 10; and m is an integer from 6 to 35.

R is preferably from 12 to 15, preferably 13 carbon atoms. $R^1$ is preferably a branched alkyl group having from 1 to 2 carbon atoms. n is preferably an integer from 1 to 5. m is preferably an integer from 8 to 25. Preferably, the weight average molecular weight of the ethoxylated alkoxylated nonionic surfactant of formula (II) is from 500 to 2000g/mol, more preferably from 600 to 1700 g/mol, most preferably 800 to 1500 g/mol.

The ethoxylated alkoxylated nonionic surfactant can be a polyoxyalkylene copolymer. The polyoxyalkylene copolymer can be a block-heteric ethoxylated alkoxylated nonionic surfactant, though block-block surfactants are preferred. Suitable polyoxyalkylene block copolymers include ethylene oxide/propylene oxide block polymers, of formula (III):

$$(EO)_x(PO)_y(EO)_x\text{, or }(PO)_x(EO)_y(PO)_x \qquad \text{Formula III}$$

wherein EO represents an ethylene oxide unit, PO represents a propylene oxide unit, and x and y are numbers detailing the average number of moles ethylene oxide and propylene oxide in each mole of product. Such materials tend to have higher molecular weights than most non-ionic surfactants, and as such can range between 1000 and 30000 g/mol, although the molecular weight should be above 2200 and preferably below 13000 to be in accordance with the invention. A preferred range for the molecular weight of the polymeric non-ionic surfactant is from 2400 to 11500 Daltons. BASF (Mount Olive, N.J.) manufactures a suitable set of derivatives and markets them under the Pluronic trademarks. Examples of these are Pluronic (trademark) F77, L62 and F88 which have the molecular weight of 6600, 2450 and 11400 g/mol respectively.

Other suitable ethoxylated alkoxylated nonionic surfactants are described in Chapter 7 of Surfactant Science and Technology, Third Edition, Wiley Press, ISBN 978-0-471-68024-6.

Most preferably the alkoxylated nonionic surfactant is selected from the group consisting of: 2-propylheptyl EO8 (Lutensol XL89-BASF); 2-propylheptyl EO5 (Lutensol XL50-BASF); $C_{10}$ alcohol EO5 (Lutensol ON 50-BASF); $C_{10}$ -alcohol EO7 (Lutensol ON 70-BASF); $C_8$-$C_{10}$ EO5 (Novel 810 FD5 Sasol); $C_{10}$ EO4 (Novel 10-4 Sasol); Tergitol 15-S-3; Tergitol 15-S-5; Tergitol 15-S-7; and Ethyl hexanol PO5EO6 (Ecosurf EH6-Dow). These surfactants have surprisingly been found to potentiate the antibacterial efficacy of the pyridinium esters.

<u>Alkyl polyglucosides</u>

Alkyl polyglycosides are biodegradable nonionic surfactants which are well known in the art and can be used in the compositions of the present invention. Suitable alkyl polyglycosides can have the general formula $C_nH_{2n+1}O(C_6H_{10}O_5)_xH$ wherein n is preferably from 8 to 16, more preferably 8 to 14, and x is at least 1. Examples of suitable alkyl polyglucoside surfactants are the TRITON™ alkyl polyglucosides from Dow; Agnique PG, Disponil APG and Glucopon alkyl polyglucosides from BASF. Preferred alkyl polyglucoside surfactants are those where n is 8 to 12, more preferably 8 to 10, such as for example Triton™ CG50 (Dow). These surfactants have surprisingly been found to enhance the antimicrobial efficacy of the pyridinium esters.

<u>Alkyl amine oxide</u>

Suitable amine oxide surfactants include: $R_1R_2R_3NO$ wherein each of $R_1$, $R_2$ and $R_3$ is independently a saturated or unsaturated, substituted or unsubstituted, linear or branched hydrocarbyl chain having from 1 to 30 carbon atoms. Preferred amine oxide surfactants are amine oxides having the following formula: $R_1R_2R_3NO$ wherein R1 is a hydrocarbyl chain comprising from 1 to 30 carbon atoms, preferably from 6 to 20, more preferably from 8 to 16 and wherein $R_2$ and $R_3$ are independently saturated or unsaturated, substituted or unsubstituted, linear or branched hydrocarbyl chains comprising from 1 to 4 carbon atoms, preferably from 1 to 3 carbon atoms, and more preferably are methyl groups. R1 may be a saturated or unsaturated, substituted or unsubstituted linear or branched hydrocarbyl chain.

Highly preferred amine oxides are $C_8$ dimethyl amine oxide, $C_{10}$ dimethyl amine oxide, $C_{12}$ dimethyl amine oxide, , $C_{14}$ dimethyl amine oxide, and mixtures thereof $C_8$ dimethyl amine oxide is commercially available under the trade name Genaminox® OC from Clariant; $C_{10}$ dimethyl amine oxide is commercially available under the trade name Genaminox® K-10 from Clariant; $C_{12}$ dimethyl amine oxide is commercially available under the trade name Genaminox® LA from Clariant and of Empigen OB from Huntsman; $C_{14}$ amine oxide is commercially available under the trade name of Empigen OH 25 from Huntsman. Other suitable amine oxide surfactants are cocoyldiethoxy amine oxide available under the trade name of Genaminox CHE from Clariant, and cocamydopropyl amine oxide commercially available under the

trade name of Empigen OS/A from Huntsman. Particularly preferred amine oxide surfactants are $C_{10}$ dimethyl amine oxide such as Genaminox K-10. These surfactants have surprisingly been found to greatly enhance the antibacterial efficacy of the pyridinium esters.

Alkyl glucamide surfactants

The composition of the invention may comprise an alkyl glucamide surfactant. Glucamide surfactants are non ionic surfactants in which the hydrophilic moiety (an amino-sugar derivative) and the hydrophobic moiety (a fatty acid) are linked via amide bonds. This results in a chemical linkage, which is highly stable under alkaline conditions. Particularly preferred alkyl glucamide surfactants are N-alkyl-N-acylglucamides of the formula (IV):

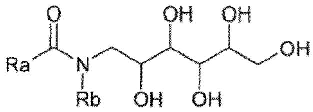

(IV)

wherein Ra is a linear or branched, saturated or unsaturated hydrocarbyl group having 6 to 22 carbon atoms, and Rb is a $C_1$-$C_4$ alkyl group. Particularly preferably, Rb in formula (III) is a methyl group. Non-limiting examples of these glucamide surfactants are: N-octanoyl-N-methylglucamide, N-nonanoyl-N-methylglucamide, N-decanoyl-N-methylglucamide, N-dodecanoyl-N-methylglucamide, N-cocoyl-N-methylglucamide, available under the trade name of GlucoPure Foam from Clariant, N-lauroyl/myristoyl-N-methylglucamide, (available under the trade name of GlucoPure Deg from Clariant, and N-octanoyl/decanoyl-N-methylglucamide, available under the trade name of GlucoPure Wet by Clariant.

Alkyl glucamine surfactants are suitable for the composition of the invention.

These surfactants are described in EP16184415 and US20190055496.

Zwitterionic and amphoteric surfactants

The hard surface cleaning composition may comprise an amphoteric surfactant, a zwitterionic surfactant, and mixtures thereof. Suitable zwitterionic surfactants typically contain both cationic and anionic groups in substantially equivalent proportions so as to be electrically neutral at the pH of use, and are well known in the art. Some common examples of zwitterionic surfactants are described in US. Pat. Nos. 2,082,275, 2,702,279 and 2,255,082.

Suitable zwitteronic surfactants include betaines such alkyl betaines, alkylamidobetaine, amidazoliniumbetaine, sulfobetaine (INCI Sultaines) as well as the phosphobetaine.

Suitable betaines are the alkyl betaines of the formula (Va), the alkyl amido betaine of the formula (Vb), the sulfo betaines of the formula (Vc) and the amido sulfobetaine of the formula (Vd);

R1-N+(CH3)2-CH2COO- (Va)

R1-CO-NH(CH2)3-N+(CH3)2-CH2COO- (Vb)

R1-N+(CH3)2-CH2CH(OH)CH2SO3- (Vc)

R1-CO-NH-(CH2)3-N+(CH3)2-CH2CH(OH)CH2SO3- (Vd)

in which R1 is a saturated or unsaturated $C_6$-$C_{22}$ alkyl residue, preferably $C_8$-$C_{18}$ alkyl residue. Particularly preferred are betaines of the formula Va such as for example N-alkyl-N-dimethyl betaine like the one sold under the trade name of Empigen® BB by Huntsman.

If the composition comprises a zwitterionic surfactant, it is preferably a betaine of the formula Va such as for example N-alkyl-N-dimethyl betaine like the one sold under the trade name of Empigen BB by Huntsman. It has been found these betaines greatly increase the antibacterial efficacy of the pyridinium esters.

Amphoteric surfactants can be either cationic or anionic depending upon the pH of the composition. Suitable amphoteric surfactants include the products sold under the trade name Miranol by Solvay-Novecare such as, for example, sodium lauroamphoacetate (Miranol Ultra L-32E), sodium stearoampho acetate (Miranol DM), disodium cocoamphodiacetate (Miranol C2m Conc NP), disodium lauroamphodiacetate (Miranol BM Conc), disodium capryloampho dipropionate (Miranol JBS), sodium mixed $C_8$ amphocarboxylate (Miranol JEM Conc), and sodium capryloampho hydroxypropyl sulfonate (Miranol JS). Other non-limiting examples of suitable amphoteric surfactants are disodium capryloamphodiacetate (Mackam 2CY 75-Solvay Novecare), octyliminodipropionate (Ampholak YJH40-Akzo Nobel), sodium lauriminodipropionate (Mirataine H2C-HA-Solvay Novecare), and sodium lauroamphohydroxypropylsulfonate (Mackam

LS- Solvay Novecare). Amphoteric surfactants might not impact negatively the antimicrobial efficacy of the pyridinium esters.

Other suitable additional surfactants can be found in McCutcheon's Detergents and Emulsifiers, North American Ed. 1980.

Cationic Surfactant

The compositions disclosed herein may comprise a cationic surfactant. Non-limiting examples of cationic surfactants include: the quaternary ammonium surfactants, which can have up to 26 carbon atoms and may include alkoxylate quaternary ammonium (AQA) surfactants, dimethyl hydroxyethyl quaternary ammonium, and/or dimethyl hydroxyethyl lauryl ammonium chloride; polyamine cationic surfactants; cationic ester surfactants; amino surfactants, e.g., amido propyldimethyl amine (APA); and mixtures thereof.

Suitable cationic surfactants also may include alkyl pyridinium compounds, alkyl quaternary ammonium compounds, alkyl quaternary phosphonium compounds, alkyl ternary sulphonium compounds, and mixtures thereof.

Suitable cationic detersive surfactants are quaternary ammonium compounds having the general formula:

$$(R)(R_1)(R_2)(R_3)N^+ X^-$$

wherein, R is a linear or branched, substituted or unsubstituted $C_{6-18}$ alkyl or alkenyl moiety, $R_1$ and $R_2$ are independently selected from methyl or ethyl moieties, $R_3$ is independently selected from a linear or branched, substituted or unsubstituted $C_{6-18}$ alkyl or alkenyl moiety or a hydroxyl, hydroxymethyl or a hydroxyethyl moiety, X is an anion which provides charge neutrality, suitable anions include: halides, for example chloride; sulphate; and sulphonate. Suitable cationic surfactants are mono-$C_{6-18}$ alkyl mono-hydroxyethyl di-methyl quaternary ammonium chlorides. Highly suitable cationic surfactants are di-$C_{8-10}$ alkyl di-methyl quaternary ammonium chloride, mono-$C_{16}$ alkyl tri-methyl quaternary ammonium chloride, di-$C_{10-12}$ alkyl di-methyl quaternary ammonium chloride and mono-$C_{10}$ alkyl mono-hydroxyethyl di-methyl quaternary ammonium chloride.

Anionic surfactants

The composition may comprise anionic surfactants but preferably, the composition is free of anionic surfactants. By "free of anionic surfactant" is herein meant that the composition comprises less than 0.05% by weight of the composition of anionic surfactant.

Particularly preferred surfactants for use herein include nonionic surfactants, in particular branched alcohol alkoxylates, more in particular 2-ethyl hexan-1-ol ethoxylated to a degree of from 4 to 6, and propoxylated to a degree of from 4 to 6, more preferably, the alcohol is first propoxylated and then ethoxylated, and 2-alkyl-1-alkanols such as alkoxylated $C_{10}$ guerbet alcohols with 1 to 14, preferably 2 to 8, more preferably 3 to 6 ethoxylate or ethoxylate-propoxylate units. Other particularly preferred non-ionic surfactants include linear alcohol alkoxylated nonionic surfactants with $C_8$, $C_{10}$, $C_{12}$, $C_{14}$ mixtures of $C_8$ to $C_{10}$, mixtures of $C_{10}$ to $C_{12}$, mixtures of $C_{12}$ to $C_{14}$, mixtures of $C_9$ to $C_{11}$ linear alkyl chain and 10 or less ethoxylate units, preferably 3 to 9 ethoxylate units. Most preferably the alkoxylated nonionic surfactant is selected from the group consisting of: $C_{12}$-$C_{14}$ alcohol EO9 (Surfonic L 24-9), 2-propylheptyl EO8 (Lutensol XL89-BASF); 2-propylheptyl EO5 (Lutensol XL50-BASF); $C_{10}$ alcohol EO5 (Lutensol ON 50-BASF); $C_{10}$ alcohol EO7 (Lutensol ON 70-BASF); $C_8$-$C_{10}$ alcohol EO5 (Novel 810 FD5 Sasol); $C_{10}$ alcohol EO4 (Novel 10-4 Sasol); and 2-ethyl-hexanol PO5EO6 (Ecosurf EH6-Dow).

Other particularly preferred surfactants for use here in include linear amine oxide surfactants, in particular $C_8$-$C_{12}$ dimethyl amine oxide, more in particular $C_{10}$ dimethyl amine oxide; alkyldimethylbetaine surfactants, more in particular N,N-Dimethyl-N-dodecylglycine betaine (Empigen BB-Huntsman); alkyl glucamide surfactants such as N-alkyl-N-acylglucamide preferably N-decanoyl-N-methylglucamine, and the alkyl glucamide surfactants sold under the name of GlucoPure®, GlucoTain®, and GlucoWet® by Clariant; alkylpolyglucoside surfactants, more in particular $C_8$ to $C_{12}$ alkyl polyglucosides, more preferably $C_8$ to $C_{10}$ alkyl polyglucosides such as for example Triton CG50 (Dow)

These surfactants improve the antimicrobial activity of the pyridinium esters.

Non-aqueous solvent

The antimicrobial system of the composition of the present invention comprises a non-aqueous solvent, the solvent comprises carbon, hydrogen and oxygen atoms. Preferred solvents for use herein include glycol ethers, diols and mixtures thereof. Suitable diols to use herein include 1,2-hexane diol, 1,2-octanediol and 1,4 butanediol. Especially preferred for use herein is 1, 4-butanediol. The composition of the invention preferably comprises a glycol ether of Formula VI:

Formula VI = R1O(R2O)nR3

wherein R1 is a linear or branched C4, C5 or C6 alkyl, a substituted or unsubstituted phenyl, preferably n-butyl. Benzyl is one of the substituted phenyls for use herein.

R2 is ethyl or isopropyl, preferably isopropyl
R3 is hydrogen or methyl, preferably hydrogen
n is 1, 2 or 3, preferably 1 or 2

Suitable glycol ethers according to Formula 1 include ethyleneglycol n-butyl ether, diethyleneglycol n-butyl ether, triethyleneglycol n-butyl ether, propyleneglycol n-butyl ether, dipropyleneglycol n-butyl ether, tripropyleneglycol n-butyl ether, ethyleneglycol n-pentyl ether, diethyleneglycol n-pentyl ether, triethyleneglycol n-pentyl ether, propyleneglycol n-pentyl ether, dipropyleneglycol n-pentyl ether, tripropyleneglycol n-pentyl ether, ethyleneglycol n-hexyl ether, diethyleneglycol n-hexyl ether, triethyleneglycol n-hexyl ether, propyleneglycol n-hexyl ether, dipropyleneglycol n-hexyl ether, tripropyleneglycol n-hexyl ether, ethyleneglycol phenyl ether, diethyleneglycol phenyl ether, triethyleneglycol phenyl ether, propyleneglycol phenyl ether, dipropyleneglycol phenyl ether, tripropyleneglycol phenyl ether, ethyleneglycol benzyl ether, diethyleneglycol benzyl ether, triethyleneglycol benzyl ether, propyleneglycol benzyl ether, dipropyleneglycol benzyl ether, tripropyleneglycol benzyl ether, ethyleneglycol isobutyl ether, diethyleneglycol isobutyl ether, triethyleneglycol isobutyl ether, propyleneglycol isobutyl ether, dipropyleneglycol isobutyl ether, tripropyleneglycol isobutyl ether, ethyleneglycol isopentyl ether, diethyleneglycol isopentyl ether, triethyleneglycol isopentyl ether, propyleneglycol isopentyl ether, dipropyleneglycol isopentyl ether, tripropyleneglycol isopentyl ether, ethyleneglycol isohexyl ether, diethyleneglycol isohexyl ether, triethyleneglycol isohexyl ether, propyleneglycol isohexyl ether, dipropyleneglycol isohexyl ether, tripropyleneglycol isohexyl ether, ethyleneglycol n-butyl methyl ether, diethyleneglycol n-butyl methyl ether triethyleneglycol n-butyl methyl ether, propyleneglycol n-butyl methyl ether, dipropyleneglycol n-butyl methyl ether, tripropyleneglycol n-butyl methyl ether, ethyleneglycol n-pentyl methyl ether, diethyleneglycol n-pentyl methyl ether, triethyleneglycol n-pentyl methyl ether, propyleneglycol n-pentyl methyl ether, dipropyleneglycol n-pentyl methyl ether, tripropyleneglycol n-pentyl methyl ether, ethyleneglycol n-hexyl methyl ether, diethyleneglycol n-hexyl methyl ether, triethyleneglycol n-hexyl methyl ether, propyleneglycol n-hexyl methyl ether, dipropyleneglycol n-hexyl methyl ether, tripropyleneglycol n-hexyl methyl ether, ethyleneglycol phenyl methyl ether, diethyleneglycol phenyl methyl ether, triethyleneglycol phenyl methyl ether, propyleneglycol phenyl methyl ether, dipropyleneglycol phenyl methyl ether, tripropyleneglycol phenyl methyl ether, ethyleneglycol benzyl methyl ether, diethyleneglycol benzyl methyl ether, triethyleneglycol benzyl methyl ether, propyleneglycol benzyl methyl ether, dipropyleneglycol benzyl methyl ether, tripropyleneglycol benzyl methyl ether, ethyleneglycol isobutyl methyl ether, diethyleneglycol isobutyl methyl ether, triethyleneglycol isobutyl methyl ether, propyleneglycol isobutyl methyl ether, dipropyleneglycol isobutyl methyl ether, tripropyleneglycol isobutyl methyl ether, ethyleneglycol isopentyl methyl ether, diethyleneglycol isopentyl methyl ether, triethyleneglycol isopentyl methyl ether, propyleneglycol isopentyl methyl ether, dipropyleneglycol isopentyl methyl ether, tripropyleneglycol isopentyl methyl ether, ethyleneglycol isohexyl methyl ether, diethyleneglycol isohexyl methyl ether, triethyleneglycol isohexyl methyl ether, propyleneglycol isohexyl methyl ether, dipropyleneglycol isohexyl methyl ether, tripropyleneglycol isohexyl methyl ether, and mixtures thereof.

Preferred glycol ether solvents according to Formula VI are ethyleneglycol n-butyl ether, diethyleneglycol n-butyl ether, triethyleneglycol n-butyl ether, propyleneglycol n-butyl ether, dipropyleneglycol n-butyl ether, tripropyleneglycol n-butyl ether, and mixtures thereof.

The most preferred glycol ether for use herein are selected from is the group consisting of of diethylene glycol butyl ether, di(propylene glycol) methyl ether and mixtures thereof.

The composition of the invention preferably comprises from about 0.1% to about 10%, more preferably from about 0.2 to about 6% by weight of the composition of the glycol ether, more preferably from about 0.2 to about 3% by weight of the composition of diethylene glycol butyl ether, di(propylene glycol) methyl ether and mixtures thereof.

<u>Optional ingredients</u>

*Chelating agent*

The antimicrobial composition can comprise a chelating agent. Suitable chelating agents, in combination with the surfactant system, improve the shine benefit. Chelating agent can be incorporated into the compositions in amounts ranging from 0.02% to 5.0%, preferably from 0.1% to 3.0%, more preferably from 0.2% to 2.0% and most preferably from 0.2% to 0.4% by weight of the composition.

Suitable phosphonate chelating agents include ethylene diamine tetra methylene phosphonates, and diethylene

triamine penta methylene phosphonates (DTPMP), and can be present either in their acid form or as salts.

A preferred biodegradable chelating agent for use herein is ethylene diamine N,N'-disuccinic acid, or alkali metal, or alkaline earth, ammonium or substitutes ammonium salts thereof or mixtures thereof. A more preferred biodegradable chelating agent is L-glutamic acid N,N-diacetic acid (GLDA) commercially available under tradename Dissolvine 47S from Akzo Nobel.

Suitable amino carboxylates include ethylene diamine tetra acetates, diethylene triamine pentaacetates, diethylene triamine pentaacetate (DTPA), N- hydroxyethylethylenediamine triacetates, nitrilotriacetates, ethylenediamine tetrapropionates, triethylenetetraaminehexa-acetates, ethanoldiglycines, and methyl glycine diacetic acid (MGDA), both in their acid form, or in their alkali metal, ammonium, and substituted ammonium salt forms. Particularly suitable amino carboxylate to be used herein is propylene diamine tetracetic acid (PDTA) which is, for instance, commercially available from BASF under the trade name Trilon FS® and methyl glycine di-acetic acid (MGDA). Most preferred aminocarboxylate used herein is diethylene triamine pentaacetate (DTPA) from BASF. Further carboxylate chelating agents for use herein include salicylic acid, aspartic acid, glutamic acid, glycine, malonic acid or mixtures thereof.

Suitable polycarboxylates include itaconic acid and sodium polyitaconate which is, for instance, commercially available from Itaconix under the trade name of Itaconix® DSP 2K™, and Itaconix® CHT121™.

*Polymers*

The antimicrobial composition may comprise an additional polymer. It has been found that the presence of a specific polymer as described herein, when present, allows further improving the grease removal performance of the composition due to the specific sudsing/foaming characteristics they provide to the composition. Suitable polymers for use herein are disclosed in EP2272942 and EP2025743.

The polymer can be selected from the group consisting of: a vinylpyrrolidone homopolymer (PVP); a polyethyleneglycol dimethylether (DM-PEG); a vinylpyrrolidone/dialkylaminoalkyl acrylate or methacrylate copolymers; a polystyrenesulphonate polymer (PSS); a poly vinyl pyridine-N-oxide (PVNO); a polyvinylpyrrolidone/ vinylimidazole copolymer (PVP-VI); a polyvinylpyrrolidone/ polyacrylic acid copolymer (PVP-AA); a polyvinylpyrrolidone/ vinylacetate copolymer (PVP-VA); a polyacrylic polymer or polyacrylicmaleic copolymer; and a polyacrylic or polyacrylic maleic phosphono end group copolymer; and mixtures thereof.

Typically, the antimicrobial hard surface cleaning composition may comprise from 0.005% to 5.0%, preferably from 0.10% to 4.0%, more preferably from 0.1% to 3.0% and most preferably from 0.20% to 1.0% by weight of the composition of said polymer.

*Thickener*

The antimicrobial composition of the invention can further comprise a thickener. Suitable thickeners herein include polyacrylate based polymers, preferably hydrophobically modified polyacrylate polymers; amide polymers; hydroxyl ethyl cellulose, preferably hydrophobically modified hydroxyl ethyl cellulose, xanthan gum, hydrogenated castor oil (HCO) and mixtures thereof.

*Other optional ingredients*

The composition of the invention may comprise a variety of other optional ingredients depending on the technical benefit aimed for and the surface treated. Suitable optional ingredients for use herein include perfume, builders, other polymers, buffers, hydrotropes, colorants, stabilisers, radical scavengers, abrasives, soil suspenders, brighteners, anti-dusting agents, dispersants, dye transfer inhibitors, pigments, silicones and/or dyes.

A preferred composition according to the invention comprises

a) from 0.01% to 5% by weight of the composition of a non-ionic surfactant selected from the group consisting of alkyl polyglucoside, alkoxylated alcohol and mixtures thereof;
b) from 0.01% to 10% by weight of the composition of a solvent is selected from the group consisting of diethylene glycol butyl ether, di(propylene glycol) methyl ether and mixtures thereof.;
c) from 0.001% to 1% by weight of the composition of a pyridinium ester according to Formula I, preferably wherein one of R2 and R3 is H and the other is Formula III, all R4 are H, R1 and R5 are independently selected from a branched or unbranched $C_6$-$C_8$ saturated hydrocarbyl groups; and
d) at least 50% by weight of the composition of water.

A preferred composition according to the invention comprises

a) from 0.01% to 5% by weight of the composition of a non-ionic surfactant comprising a fatty alcohol preferably an alkoxylated alcohol comprising from 6 to 16 carbon atoms and from 2 to 12 alkoxy groups, preferably from 4 to 10 ethoxy groups or a branched ethoxylated propoxylated alcohol;
b) from 0. 01% to 10% by weight of the composition of a solvent is selected from the group consisting of glycol ethers, diols and mixtures thereof;
c) from 0.001 to 1% by weight of the composition of a pyridinium ester according to Formula I, preferably wherein one of R2 and R3 is H and the other is Formula III, all R4 are H, R1 and R5 are independently selected from a branched or unbranched $C_6$-$C_8$ saturated hydrocarbyl groups;
d) from 85% to 98% by weight of the composition of water.

A preferred composition according to the invention comprises

a) from 0.01% to 5% by weight of the composition of a non-ionic surfactant selected from the group consisting of alkyl polyglucoside, alkoxylated alcohols and mixtures thereof;
b) from 0. 01% to 10% by weight of the composition of a solvent is selected from the group consisting of diethylene glycol butyl ether, di(propylene glycol) methyl ether and mixtures thereof.;
c) from 0.001 to 1% by weight of the composition of a pyridinium ester according to Formula I, preferably wherein one of R2 and R3 is H and the other is Formula III, all R4 are H, R1 and R5 are independently selected from a branched or unbranched $C_6$-$C_8$ saturated hydrocarbyl groups;
d) from 85% to 98% by weight of the composition of water.

Wipe

The present invention also relates to an article treated with the composition of the invention. The article is preferably a wipe. Suitable wipes can be fibrous. Suitable fibrous wipes can comprise polymeric fibres, cellulose fibres, and combinations thereof. Suitable cellulose-based wipes include kitchen wipes, and the like. Suitable polymeric fibres include polyethylene, polyester, and the like. Polymeric fibres can be spun-bonded to form the wipe. Methods for preparing thermally bonded fibrous materials are described in U.S. application Ser. No. 08/479,096 (see especially pages 16-20) and U.S. Pat. No. 5,549,589 (see especially Columns 9 to 10). Suitable pads include foams and the like, such as HIPE-derived hydrophilic, polymeric foam. Such foams and methods for their preparation are described in U.S. Pat. No. 5,550,167; and U.S. patent application Ser. No. 08/370,695.
The load factor is defined as the weight ratio of antimicrobial solution to nonwoven substrate is from about 3X to about 10X. Preferably, the load factor is between 4X and 8X, or from 4.5X to 7.5X, or from 5X to 7X. It is found that higher load factors for pre-moistened wipes in accordance with the invention are preferable since they help increase product mileage.

Method of cleaning a surface

The cleaning composition of the invention is particularly suited for cleaning surfaces selected from the group consisting of: ceramic, enamel, stainless steel, Inox®, Formica®, vinyl, no-wax vinyl, linoleum, melamine, glass, plastics and plastified wood, and combinations thereof. In particular, the compositions are particularly suited for reducing the microbial population, while leaving surfaces clean, shiny and grease free.
The compositions described herein can be used neat or can be achieved by diluting with water a concentrated composition prior to applying to the surface. In preferred methods, the hard surface cleaning composition is applied neat, more preferably, the hard surface cleaning composition is sprayed onto the hard surface.
The composition can be applied by any suitable means, including using a mop, sponge, cloth, paper towel, or other suitable implement.
The surface may be rinsed, preferably with clean water, in an optional further step, and also as a further step, wiped, such as with a cloth or a paper towel.
In another preferred embodiment of the present invention said method of cleaning a surface includes the steps of applying, preferably spraying, said liquid composition onto said hard surface, leaving said liquid composition to act onto said surface for a period of time with or without applying mechanical action, and optionally removing said liquid composition, preferably removing said liquid composition by rinsing said hard surface with water and/or wiping said hard surface with an appropriate implement, e.g., a sponge, a paper or cloth towel and the like. Such compositions are often referred to as "ready-to-use" compositions. In preferred methods, the surface is not rinsed after application of the antimicrobial composition.
It is believed that antimicrobial compositions comprising specific surfactants, non-aqueous solvents and the pyridinium ester of Formula I deliver very good antimicrobial efficacy at very low levels of antimicrobial agent. The

antimicrobial hard surface cleaning composition of the present invention exhibits improved antimicrobial efficacy, good grease cleaning and/or streak-free shine.

TEST METHODS

pH measurement

The pH is measured on the neat composition, at 25°C, using a pH meter with compatible gel-filled pH probe (such as Sartarius PT-10P meter with Toledo probe part number 52 000 100), calibrated according to the instruction manual.

Surface Antimicrobial Kill Testing Method

Inoculum is prepared by streaking microorganism onto a Tryptic Soy Agar (TSA) plate and incubating for 24 h at 33°C. Plated bacteria is resuspended in saline until the transmittance percentage falls within the accepted range for ~$10^8$ cfu/mL. For K. pneumonia (Kp) and *P. aeruginosa* (Pa), this is 31.00-33.00 %T. For *S. aureus* (Sa) this is 23.00-25.00 %T. A 10x concentration is made by spinning down the prepared inoculum, removing the supernatant saline from the bacterial pellet, then resuspending the pellet in 1/10th of the original volume of saline. The final inoculum is prepared with 5% of "soil" in the form of Fetal Bovine Serum (FBS).

A 18x18 mm sterile glass coverslip is placed into an appropriate container (e.g. petri dish). 20 µL of prepared inoculum is added to the glass coverslip. Inoculum is then air-dried for 30 minutes in a 36°C incubator. After drying, 40 µL of test sample is added on top of the dried inoculum. This is incubated at room temperature for the desired contact time. 5 mL of neutralizing media (Modified Letheen Broth + Tween + Lecithin (MLBTL)) is added to the coverslip and shaken on an orbital shaker for 5 minutes to ensure proper neutralization and extraction. Viability is then assessed of the neutralized and extracted sample by traditional/altemative microbiological techniques.

Normalization of Antimicrobial Activity Method

The normalized antimicrobial activity reported in the performance examples below was generated from detection time data obtained as described in the Surface Antimicrobial Kill Test Method. To account for day-to-day variability an internal standard was tested with each series. The internal standard was a composition of 0.25 wt% Triclosan, Sigma Aldrich (St. Louis, MO, USA; CAS # 3380-34-5, 97.0-103.0% (active substance, GC)), 50 wt% DMSO, and 49.75 wt% $H_2O$, with a final solution pH of 7.7. Normalized activity is reported following the equation 1 below.

$$\text{Normalized Activity}=(\alpha\Delta DT-\beta\Delta DT)/\ (\gamma\Delta DT-\beta\Delta DT) \qquad \text{equation 1.}$$

where, $\alpha\Delta DT$ is the average detection time of the antimicrobial compound, and $\gamma\Delta DT$ is the average detection time of the reference triclosan internal standard, and $\beta\Delta DT$ is the average detection time of the background unperturbed bacteria.

Formulation Method for Surface Kill Testing

Pyridinium esters of the present disclosure were tested as aqueous solutions in the Surface Antimicrobial Kill Testing Method above. To prepare test solutions pyridinium esters were first dissolved in the appropriate solvent. Second the selected surfactant is added to the mixture. After vortex (1 min, Vortex-2 Genie, vortex speed 8) mixing, the samples were then diluted with ½ portion of reverse osmosis water (RO water, Millipore Direct-Q™, pH 6). The aqueous solution was then vortexed (1 min, Vortex-2 Genie, vortex speed 8), to which the second ½ portion of water is added. The pH of the solution was then adjusted to the target pH using either 1N HCl, Glacial Acetic Acid, or 1N NaOH. The final solution was then vortexed (1 min, Vortex-2 Genie, vortex speed 8). An example batch sheet is provided below in Table 1.

Surfactant description and supplier: Tween® 80 (Sigma Aldrich, St. Louis, MO, USA), Plantaren® 2000 N UP (BASF, Florham Park, NJ, USA), EcoSurf™ EH6 (Sigma Aldrich, St. Louis, MO, USA), EcoSurf™ EH9 (Sigma Aldrich, St. Louis, MO, USA), Surfonic® L24-9 (Huntsman Holland BV, Botlek-Rotterdam, Netherlands), Miranol® Ultra L-32 (Solvay S.A., Brussels, Belgium), Cetyltrimethylammonium chloride (CTAC, 25 wt% in H2O, Sigma Aldrich, St. Louis, MO, USA), Sodium Lauryl Sulfate (SLS, 29 wt% in H2O, Stepan, Northfield, IL, USA), Tergitol™ TMN-6 (Sigma Aldrich, St. Louis, MO, USA), $C_{10}$ dimethyl amine oxide is commercially available under the trade name Genaminox® K-10 from Clariant.

Solvents and supplier: 1,4-Butanediol (99%, Sigma Aldrich, St. Louis, MO, USA), Ethylene Glycol (99%, Sigma Aldrich, St. Louis, MO, USA), Diethylene glycol mono-n-butyl ether (99%, DEGBE, Thermo Fisher Scientific, Ward Hill, MA, USA), Di(propylene glycol)methyl ether, mixture of isomers (99%, DPGME, Sigma Aldrich, St. Louis, MO, USA), Dimethyl sulfoxide (99%, DMSO, igma Aldrich, St. Louis, MO, USA), 1,2-Hexanediol (98%, Sigma Aldrich, St. Louis, MO,

USA), Hexylene Glycol (99%, Sigma Aldrich, St. Louis, MO, USA), 2-Phenoxyethanol (99%, Sigma Aldrich, St. Louis, MO, USA), Di(ethylene glycol) hexyl ether (95%, Sigma Aldrich, St. Louis, MO, USA).

Table 1: Illustrative formulation sheet for hexyl 3-(alkylpyridinium)acrylate halide solutions for surface antimicrobial kill testing.

| Ingredients | Quantity (g) |
|---|---|
| Hexyl 3-(alkylpyridinium)acrylate halide | 0.05 |
| EcoSurf EH-9 | 0.1 |
| DEGBE | 0.5 |
| Reverse Osmosis Water | 9.35 |
| Total Batch Size | 10.0 |

Table 2. Illustrative formulation sheet for hexyl 3-(alkylpyridinium)acrylate halide solutions for surface antimicrobial kill testing.

| Ingredients | Quantity (g) |
|---|---|
| Hexyl 3-(alkylpyridinium)acrylate halide | 0.05 |
| EcoSurf EH-9 | 0.1 |
| 1,2-Hexanediol | 0.5 |
| Reverse Osmosis Water | 9.35 |
| Total Batch Size | 10.0 |

Table 3. Illustrative formulation sheet for hexyl 3-(alkylpyridinium)acrylate halide solutions for surface antimicrobial kill testing.

| Ingredients | Quantity (g) |
|---|---|
| Hexyl 3-(alkylpyridinium)acrylate halide | 0.05 |
| EcoSurf EH-9 | 0.1 |
| Hexylene Glycol | 0.5 |
| Reverse Osmosis Water | 9.35 |
| Total Batch Size | 10.0 |

Table 4. Illustrative formulation sheet for hexyl 3-(alkylpyridinium)acrylate halide solutions for surface antimicrobial kill testing.

| Ingredients | Quantity (g) |
|---|---|
| Hexyl 3-(alkylpyridinium)acrylate halide | 0.05 |
| Surfonic L 24-9 | 0.1 |
| Ethylene Glycol | 0.5 |
| Reverse Osmosis Water | 9.35 |
| Total Batch Size | 10.0 |

Table 5. Illustrative formulation sheet for hexyl 3-(alkylpyridinium)acrylate halide solutions for surface antimicrobial kill testing.

| Ingredients | Quantity (g) |
|---|---|
| Hexyl 3-(alkylpyridinium)acrylate halide | 0.05 |
| Tergitol TMN-6 | 0.1 |
| Di(ethylene glycol) hexyl ether | 0.5 |
| Reverse Osmosis Water | 9.35 |
| Total Batch Size | 10.0 |

Table 6. Illustrative formulation sheet for hexyl 3-(alkylpyridinium)acrylate halide solutions for surface antimicrobial kill testing.

| Ingredients | Quantity (g) |
|---|---|
| Hexyl 3-(alkylpyridinium)acrylate halide | 0.05 |
| EcoSurf EH-6 | 0.1 |
| 2-Phenoxyethanol | 0.5 |
| Reverse Osmosis Water | 9.35 |
| Total Batch Size | 10.0 |

Table 7. Illustrative formulation sheet for hexyl 3-(alkylpyridinium)acrylate halide solutions for surface antimicrobial kill testing.

| Ingredients | Quantity (g) |
|---|---|
| Hexyl 3-(alkylpyridinium)acrylate halide | 0.05 |
| EcoSurf EH-9 | 0.1 |
| 2-Phenoxyethanol | 0.1 |
| DPGME | 0.4 |
| Reverse Osmosis Water | 9.35 |
| Total Batch Size | 10.0 |

Table 8. Illustrative formulation sheet for hexyl 3-(alkylpyridinium)acrylate halide solutions for surface antimicrobial kill testing.

| Ingredients | Quantity (g) |
|---|---|
| Hexyl 3-(alkylpyridinium)acrylate halide | 0.05 |
| Genaminox | 0.1 |
| DEGBE | 0.5 |
| Reverse Osmosis Water | 9.35 |
| Total Batch Size | 10.0 |

Table 9. Illustrative formulation sheet for hexyl 3-(alkylpyridinium)acrylate halide solutions for surface antimicrobial kill testing.

| Ingredients | Quantity (g) |
|---|---|
| Hexyl 3-(alkylpyridinium)acrylate halide | 0.05 |
| Didecyldimethylammonium chloride | 0.5 |
| DEGBE | 0.5 |
| Reverse Osmosis Water | 9.4 |
| Total Batch Size | 10.0 |

Table 10. Illustrative formulation sheet for hexyl 3-(alkylpyridinium)acrylate halide solutions for surface antimicrobial kill testing.

| Ingredients | Quantity (g) |
|---|---|
| Hexyl 3-(alkylpyridinium)acrylate halide | 0.05 |
| Surfonic L 24-9 | 0.05 |
| Tween 80 | 0.05 |
| DEGBE | 0.5 |
| Reverse Osmosis Water | 9.35 |
| Total Batch Size | 10.0 |

Table 11. Illustrative formulation sheet for hexyl 3-(alkylpyridinium)acrylate halide solutions for surface antimicrobial kill testing.

| Ingredients | Quantity (g) |
|---|---|
| Hexyl 3-(alkylpyridinium)acrylate halide | 0.025 |
| EcoSurf EH-9 | 0.05 |
| DEGBE | 0.25 |
| Reverse Osmosis Water | 9.675 |
| Total Batch Size | 10.0 |

Table 12. Illustrative formulation sheet for hexyl 3-(alkylpyridinium)acrylate halide solutions for surface antimicrobial kill testing.

| Ingredients | Quantity (g) |
|---|---|
| Hexyl 3-(alkylpyridinium)acrylate halide | 0.05 |
| Sodium Lauryl Sulfate (SLS) | 0.05 |
| Tween 80 | 0.05 |
| DEGBE | 0.5 |
| Reverse Osmosis Water | 9.35 |
| Total Batch Size | 10.0 |

Synthesis & Comparative Examples

**Methods of Preparing Pyridinium esters**

In the following synthesis examples, the materials are generally obtained / available from Sigma-Aldrich (St. Louis, MO, USA), except as indicated below. The carboxaldehydes are generally provided at >97% purity. The alkyl halides are generally provided at >98% or >99% purity. Monohexylmalonate is prepared according to known procedures (European Journal of Medicinal Chemistry 46 (2011) 773-777).

**General Method A: General Preparation of Hexyl 3-(pyridyl)acrylate Compounds**

To prepare a hexyl 3-(pyridyl)acrylate, a round bottom flask is charged with 1 equiv. of a pyridine carboxaldehyde, 1.1 equiv. monohexylmalonate, 1 equiv. pyridine, and 1.6 equiv. β-alanine as catalysts. The flask is then diluted with acetonitrile and refluxed for 24 h using a dean-stark apparatus. The resulting mixture is acidified with HCl, diluted with $H_2O$ and washed with EtOAc. The aqueous layer is neutralized with sodium bicarbonate followed by extraction of the product with EtOAc. The organic phase is dried over $MgSO_4$ and filtered. The solvent is removed to yield the hexyl 3-(pyridyl) acrylate.

**Synthetic Example 1**

Synthetic Example 1, hexyl 3-(3-pyridyl)acrylate, was prepared as described in General Method A, but using 10.7 g of nicotinaldehyde, 20.7 g of hexyl malonate, 7.9 g of pyridine, and 14.3 g of β-alanine.

**Synthetic Example 2**

Synthetic Example 2, hexyl 3-(4-pyridyl)acrylate, was prepared as described in General Method A, but using 3.21 g of 4-pyridylcarboxaldehyde, 6.21 g of hexyl malonate, 2.37 g of pyridine, and 4.28 g of β-alanine.

**General Method B: General Preparation of Hexyl 3-(methylpyridinium)acrylate Iodide Compounds**

To prepare a hexyl 3-(methylpyridinium)acrylate iodide, a round bottom flask is charged with 1 equiv. of a hexyl 3-(pyridyl)acrylate and 2 equiv. methyl iodide. The flask is then diluted with acetonitrile and refluxed for 4 h. The pyridinium product is precipitated through addition of ether.

**Synthetic Example 3**

Synthetic Example 3, hexyl 3-(3-methylpyridinium)acrylate iodide, was prepared as described in General Method B, but using 4 g of Synthetic Example 1 and 4.87 g of methyl iodide. The independent solid 3 appears stable for several months by 1H NMR.

**Synthetic Example 4**

Synthetic Example 4, hexyl 3-(4-methylpyridinium)acrylate iodide, was prepared as described in General Method B, but using 4 g of Synthetic Example 2 and 4.87 g of methyl iodide. The independent solid 4 appears stable for several months by 1H NMR.

**General Method C: General Preparation of Hexyl 3-(alkylpyridinium)acrylate Bromide Compounds**

To prepare a hexyl 3-(alkylpyridinium)acrylate bromide, a round bottom flask is charged with 1 equiv. of a hexyl 3-(pyridyl)acrylate and 1.5-2 equiv. of a bromoalkane. The flask is then diluted with acetonitrile and refluxed for 3 days. The pyridinium product is precipitated through addition of ether.

**Synthetic Example 5**

Synthetic Example 5, hexyl 3-(3-hexylpyridinium)acrylate bromide, was prepared as described in General Method C, but using 2.3 g of Synthetic Example 1 and 3.0 g of bromohexane. The independent solid 5 appears stable for several months by 1H NMR.

**Synthetic Example 6**

Synthetic Example 6, hexyl 3-(4-hexylpyridinium)acrylate bromide, was prepared as described in General Method C, but using 2.3 g of Synthetic Example 2 and 3.0 g of bromohexane. The independent solid 6 appears stable for several months by 1H NMR.

**Synthetic Example 7**

Synthetic Example 7, hexyl 3-(3-octylpyridinium)acrylate bromide, was prepared as described in General Method C, but using 3.0 g of Synthetic Example 1 and 5.0 g of bromooctane. The independent solid 7 appears stable for several months by 1H NMR.

**Synthetic Example 8**

Synthetic Example 8, hexyl 3-(4-octylpyridinium)acrylate bromide, was prepared as described in General Method C, but using 2.3 g of Synthetic Example 2 and 3.0 g of bromooctane. The independent solid 8 appears stable for several months by 1H NMR.

**Structures of the Synthetic Examples.**

Table 13. Structural representation of the Synthetic Examples 1-8. Compounds 1-3 are not representative of the present invention,

| No. | Structure |
|---|---|
| 1 | 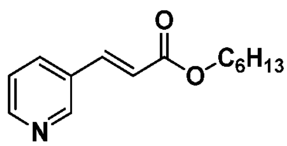 |

(continued)

| No. | Structure |
|---|---|
| 2 | 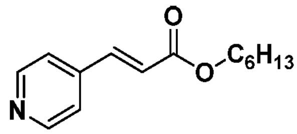 |
| 3 | 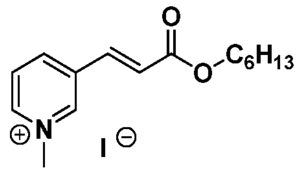 |
| 4 | 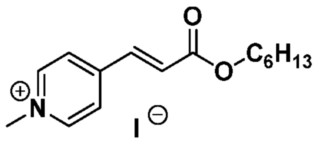 |
| 5 | 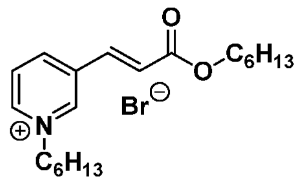 |
| 6 | 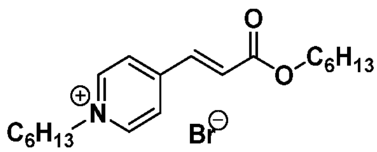 |
| 7 | 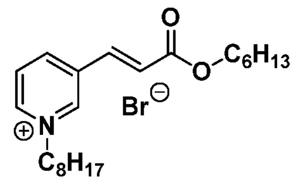 |
| 8 | 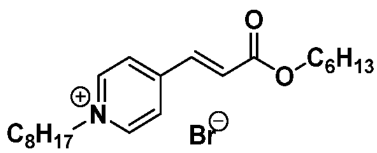 |

## Performance Examples

In Performance Examples 1-4 below, formulations comprising solvent, surfactant, and pyridinium esters according to the present disclosure (e.g., based on hexyl 3-(alkylpyridinium)acrylate halides), are evaluated via microbial surface kill tests according to the Surface Antimicrobial Kill Testing Method provided above. After treatment, microbial growth is referenced and reported to a triclosan sample and unaltered microbes according to the Normalization of Antimicrobial Activity provided above. Therefore, values greater than one would be more active above the internal triclosan control, with values less than one but greater than zero still having antimicrobial activity. The data below shows the benefits afforded by hexyl 3-(alkylpyridinium)acrylate halides.

**Performance Example 1.** Antimicrobial Activity of Formulations of Synthetic Examples 1-8.

Table 14. Antimicrobial Activity of Formulations of Synthetic Examples.

| Antimicrobial Compound (0.5 wt%)α | Gram Positive Referenced Microbial Activity |
|---|---|
| Synthetic Example 1 | 0.11 |
| Synthetic Example 3 | 0.21 |
| Synthetic Example 4 | 1.2 |
| Synthetic Example 5 | >1.42 |
| Synthetic Example 6 | >1.39 |
| Synthetic Example 7 | >1.42 |
| Synthetic Example 8 | >1.33 |

αRemaining wt% fraction composed of 3 wt% DEGBE, 1 wt% EcoSurf EH-9, 95.5 wt% reverse osmosis water at pH 7.

As shown in Table 14, alkylation of the pyridyl structure is necessary for antimicrobial activity against staphylococcus aureus. A strong preference is given towards $C_6$-$C_8$ carbon chain lengths. Location of the pyridinium in the para position relative to the acrylate provides higher antimicrobial efficacy with shorter chain lengths while the pyridinium in the meta position is more effective with longer chain lengths.

**Performance Example 2.** Examination of surfactant on surface antimicrobial efficacy of Synthetic Example 5.

Table 15. Examination of surfactant on surface antimicrobial efficacy of Synthetic Example 5.

| Row | Surfactant (1 wt%)α | % Synthetic Example 5 | Gram Negative Referenced Microbial Activity | Gram Positive Referenced Microbial Activity |
|---|---|---|---|---|
| 1 | EH6 | - | 0.18 | 0.07 |
| 2 | L24-9 | - | -0.06 | 0.06 |
| 3 | Tween80 | - | -0.10 | 0.01 |
| 4 | Miranol Ultra | - | 0.36 | 0.10 |
| 5 | Plantaren 2000 | - | 0.33 | 0.23 |
| 6 | Crodasinic LS30 | - | 0.16 | 0.29 |
| 7 | SLS | - | 0.13 | 0.22 |
| 8 | EH6 | 0.25 | >2.58 | 1.09 |
| 9 | L24-9 | 0.25 | 1.34 | 0.54 |
| 10 | Tween80 | 0.25 | 1.37 | 0.57 |
| 11 | Miranol Ultra | 0.25 | 1.92 | 0.78 |
| 12 | Plantaren 2000 | 0.25 | >2.58 | > 2.33 |
| 13 | Crodasinic LS30 | 0.25 | 0.41 | 0.42 |
| 14 | SLS | 0.25 | 0.08 | 0.20 |

α Remaining wt% fraction composed of 3 wt% DEGBE, 95.75 wt% reverse osmosis water at pH 7.

As shown in table 15, formulations containing Tween® 80, Plantaren® 2000 N UP, EcoSurf™ EH6, EcoSurf™ EH9, Surfonic® L24-9, or Miranol® Ultra L-32 all show an improvement in microbial activity over the surfactant, solvent, chassis alone. Combining Synthetic Example 5 with APG's, alcohol ethoxylates, and amphoteric surfactants resulted in measurable microbial activity. A preference is noted for APG's, row 12, with strong performance in gram-negative and gram-positive bacteria. Within alcohol ethoxylate surfactants a preference is given for shorter chain alcohols. Compositions containing anionic surfactants illustrated with sodium lauryl sulfate and Crodasinic LS30 had reduced activity. Without wishing to be bound by theory we asses this reduction in activity to ion paring.

**Performance Example 3.** Examination of pH on surface antimicrobial efficacy of Synthetic Example 4.

Table 16. Examination of pH on surface antimicrobial efficacy of Synthetic Example 4.

| pHα | Gram Positive Referenced Microbial Activity |
|---|---|
| 3 | >2.58 |
| 7 | >2.8 |

(continued)

| pH^α | Gram Positive Referenced Microbial Activity |
|---|---|
| 9 | 2.53 |

^α Formulations composed of 0.5 wt% Synthetic Example 5, 1 wt% EcoSurf EH9, 3 wt% DEGBE, 95.5 wt% reverse osmosis water. pH adjusted with either HCl or NaOH.

As shown in table 16, Synthetic Example 5 maintains antimicrobial efficacy in formulations of pH ranging from at least 3-9.

**Performance Example 4.** Examination of concentration on surface antimicrobial efficacy of Synthetic Example 5.

Table 17. Examination of concentration on surface antimicrobial efficacy of Synthetic Example 5.

| Synthetic Example 5 wt%^α | Gram Positive Referenced Microbial Activity |
|---|---|
| 0.5 | >2.87 |
| 0.25 | 2.02 |
| 0.125 | 0.93 |
| 0.063 | 0.55 |

^α Formulations composed of 1 wt% EcoSurf EH9, 3 wt% DEGBE, and remaining wt% fraction composed of water at pH 7.

As shown in table 17, Synthetic Example 5 maintains antimicrobial efficacy against staphylococcus aureus in concentrations down to 0.063 wt%.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A pyridinium ester according to Formula I

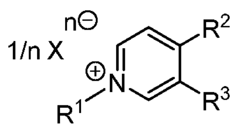

Formula I,

wherein 1/n $X^{n-}$ is a counterion and n is 1 or 2;
wherein one of $R^2$ and $R^3$ is H and the other is selected from the group consisting of Formula II, Formula III and Formula IV

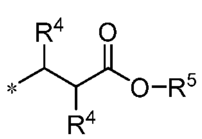

Formula II

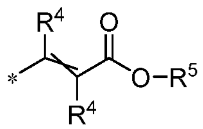

Formula III

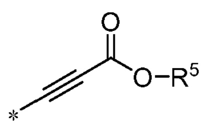

Formula IV

wherein * represents the point of attachment to the heteroaryl ring,

wherein Formula III is selected from the group consisting of a *Z*- configuration, an *E*-configuration, and mixtures thereof, preferably an E-configuration,
wherein each $R^4$ is independently selected from hydrogen and a $C_1$-$C_4$ hydrocarbyl group

and
wherein $R^5$ is a branched or unbranched, saturated or unsaturated, $C_4$-$C_{12}$ hydrocarbyl group, preferably $R^5$ is a branched or unbranched $C_6$-$C_8$ saturated hydrocarbyl group

wherein when $R^2$ is H, $R^1$ is a branched or unbranched, saturated or unsaturated, $C_4$-$C_{12}$ hydrocarbyl group; when $R^3$ is H, $R^1$ is a branched or unbranched, saturated or unsaturated, $C_1$-$C_{12}$ hydrocarbyl group.

2. A pyridinium ester according to claim 1 wherein one of $R^2$ and $R^3$ is H and the other is Formula III, preferably Formula III is

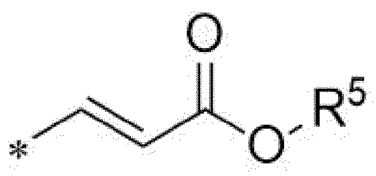

more preferably, $R^5$ is selected from a branched or unbranched $C_6$-$C_8$ saturated hydrocarbyl group.

3. A pyridinium ester according to any of claims 1 or 2 wherein the pyridinium ester is selected from the group consisting of:

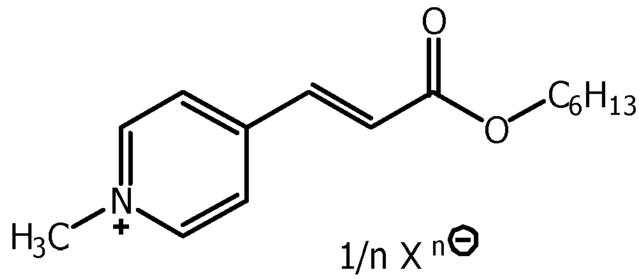

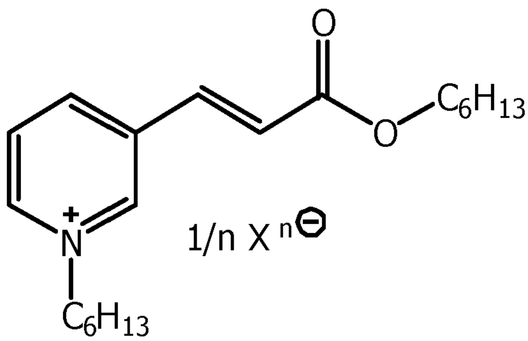

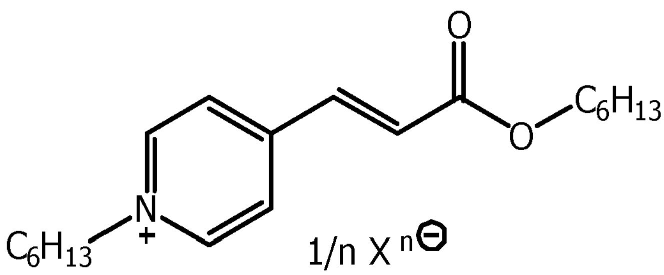

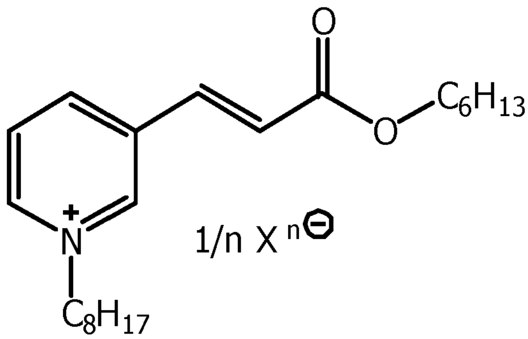

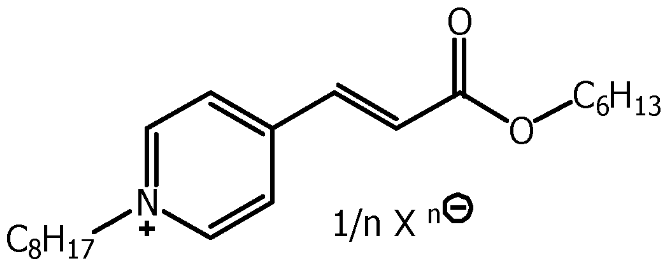

and a mixture thereof.

**4.** An antimicrobial composition comprising an antimicrobial system, the antimicrobial system comprising:

a. a surfactant;
b. a solvent; and
c. a pyridinium ester according to any of the preceding claims.

**5.** An antimicrobial composition according to the preceding claim wherein antimicrobial system comprises:

a. from about 0.01 to about 10% by weight of the composition of a surfactant selected from the group consisting of non-ionic surfactant, cationic surfactant, amphoteric surfactant and a mixture thereof;
b. preferably from about 0.01 to about 10% by weight of the composition of a non-aqueous solvent;
c. a pyridinium ester according to any of claims 1 to 3, preferably a pyridinium ester according to claim 3; and optionally

at least 50% by weight of the composition of water;

**6.** An antimicrobial composition according to any of claims 4 or 5 wherein the composition comprises a non-ionic surfactant or amphoteric surfactant selected from the group consisting of alkyl polyglucoside, alkoxylated alcohol, alkyl betaine, alkyl glucamine, alkyl glucamide, alkyl amine oxide and mixtures thereof, preferably the composition comprises a non-ionic surfactant selected from the group consisting of alkyl polyglucoside, alkoxylated alcohol and mixtures thereof.

**7.** An antimicrobial composition according to the preceding claim wherein the non-ionic surfactant comprises an alkyl polyglucoside.

**8.** An antimicrobial composition according to any of claims 4 to 7 wherein the non-aqueous solvent is selected from the group consisting of glycol ethers, diols and mixtures thereof, preferably the non-aqueous solvent is selected from the group consisting of diethylene glycol butyl ether, di(propylene glycol) methyl ether and mixtures thereof.

**9.** An antimicrobial composition according to any of claims 4 to 8 comprising:

a. from about 0.01 to about 10% by weight of the composition of a of non-ionic surfactant selected from the group consisting of alkyl polyglucoside, alkoxylated alcohol and mixtures thereof; and
b. from about 0.01 to about 10% by weight of the composition of a non-aqueous solvent selected from the group

consisting of diethylene glycol butyl ether, di(propylene glycol) methyl ether and mixtures thereof.

**10.** An antimicrobial composition according to any of claims 4 to 9 wherein the composition comprises at least 80% by weight of the composition of water.

**11.** An antimicrobial composition according to any of the claims 4 to 10 wherein the composition is in the form of a clear liquid.

**12.** An antimicrobial composition according to any of claims 4 to 11 wherein the composition is in the form of a ready-to-use spray.

**13.** An antimicrobial composition according to any of claims 4 to 12, wherein the composition comprises an additional benefit agent selected from the group consisting of a perfume raw material including aldehydes and/or ketones, an additional antimicrobial agent, a pesticide, an insect repellant, an anti-fungal agent, an antiviral agent, a herbicidal agent, a hueing dye, an antioxidant, a non-perfume organoleptic, a polymer, an abrasive, a stabilizer, a bitterant, a microcapsule or a combination thereof.

**14.** An article treated with an antimicrobial composition according to any of claims 4 to 12 wherein the article is in the form of a disposable or partially reusable substrate comprising one or more nonwoven layers and the substrate has a load factor of from about 3 times to about 10 times of composition per gram of nonwoven substrate.

**15.** A method of sanitizing an inanimate surface comprising the step of contacting the surface with an antimicrobial composition or article according to any of claims 4 to 14.

**16.** Use of a composition or article according to any of claims 4 to 14 to provide antimicrobial benefits to an inanimate surface.

**Patentansprüche**

**1.** Pyridiniumester nach Formel I

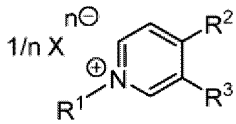

Formel I,

wobei 1/n $X^{n-}$ ein Gegenion ist und n 1 oder 2 ist;
wobei eines von $R^2$ und $R^3$ H ist und das andere aus der Gruppe ausgewählt ist, bestehend aus Formel II, Formel III und Formel IV

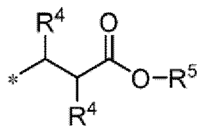

Formel II

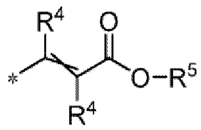

Formel III

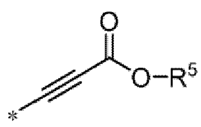

Formel IV

wobei * den Anknüpfungspunkt an den Heteroarylring darstellt,
wobei Formel III aus der Gruppe ausgewählt ist, bestehend aus einer *Z*-Konfiguration, einer *E*-Konfiguration und Mischungen davon, vorzugsweise einer *E*-Konfiguration,
wobei jedes $R^4$ unabhängig aus Wasserstoff und einer $C_1$-$C_4$-Hydrocarbylgruppe ausgewählt ist
und
wobei $R^5$ eine verzweigte oder unverzweigte, gesättigte oder ungesättigte $C_4$-$C_{12}$-Hydrocarbylgruppe ist, vorzugsweise $R^5$ eine verzweigte oder unverzweigte gesättigte $C_6$-$C_8$-Hydrocarbylgruppe ist
wobei, wenn $R^2$ H ist, $R^1$ eine verzweigte oder unverzweigte, gesättigte oder ungesättigte $C_4$-$C_{12}$-Hydrocarbylgruppe ist;

wenn $R^3$ H ist, $R^1$ eine verzweigte oder unverzweigte, gesättigte oder ungesättigte $C_1$-$C_{12}$-Hydrocarbylgruppe ist.

**2.** Pyridiniumester nach Anspruch 1, wobei eines von $R^2$ und $R^3$ H ist und das andere Formel III ist, vorzugsweise Formel III ist

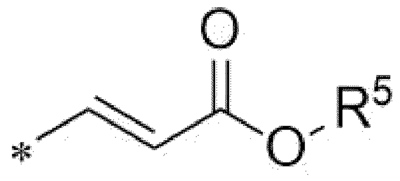

stärker bevorzugt $R^5$ ausgewählt ist aus einer verzweigten oder unverzweigten gesättigten $C_6$-$C_8$-Hydrocarbylgruppe.

**3.** Pyridiniumester nach einem der Ansprüche 1 oder 2, wobei das Pyridinium aus der Gruppe ausgewählt ist, bestehend aus:

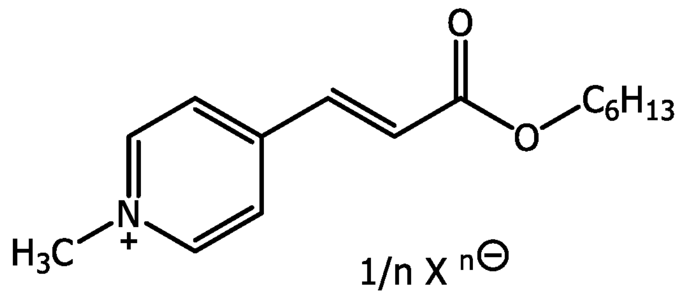

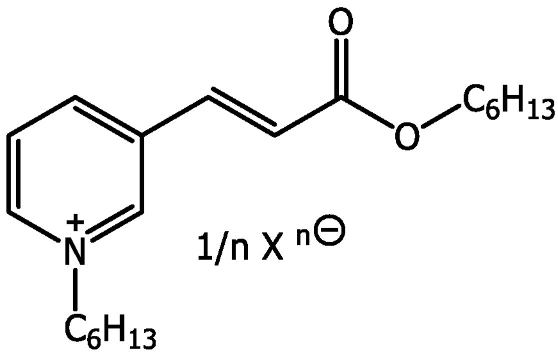

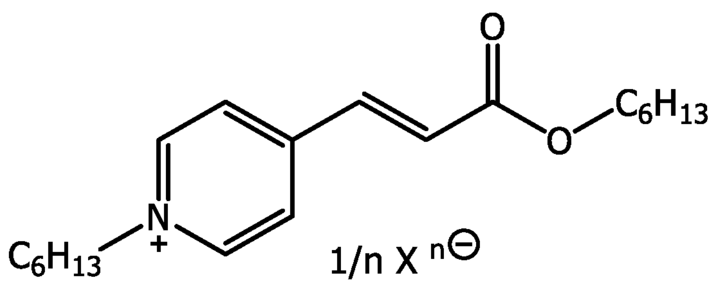

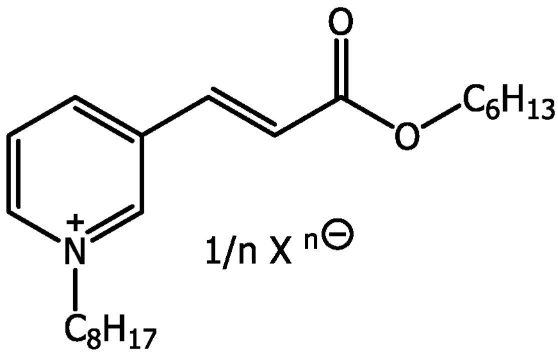

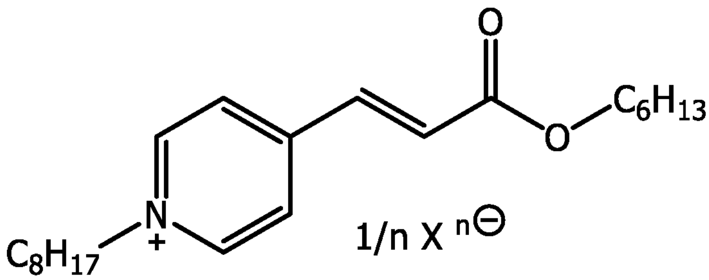

und einer Mischung davon.

4. Antimikrobielle Zusammensetzung, umfassend ein antimikrobielles System, das antimikrobielle System umfassend:

   a. ein Tensid;
   b. ein Lösungsmittel; und
   c. Pyridiniumester nach einem der vorstehenden Ansprüche.

5. Antimikrobielle Zusammensetzung nach dem vorstehenden Anspruch, wobei das antimikrobielle System umfasst:

   a. zu etwa 0,01 bis etwa 10 Gew.-% der Zusammensetzung ein Tensid, das aus der Gruppe ausgewählt ist, bestehend aus nichtionischem Tensid, kationenaktivem Tensid, amphoterem Tensid und einer Mischung davon;
   b. vorzugsweise zu etwa 0,01 bis etwa 10 Gew.-% der Zusammensetzung ein nichtwässriges Lösungsmittel;
   c. ein Pyridiniumester nach einem der Ansprüche 1 bis 3, vorzugsweise ein Pyridiniumester nach Anspruch 3; und optional

   zu mindestens 50 Gew.-% der Zusammensetzung Wasser;

6. Antimikrobielle Zusammensetzung nach einem der Ansprüche 4 oder 5, wobei die Zusammensetzung ein nichtionisches Tensid oder amphoteres Tensid umfasst, das aus der Gruppe ausgewählt ist, bestehend aus Alkylpolyglucosid, alkoxyliertem Alkohol, Alkylbetain, Alkylglucamin, Alkylglucamid, Alkylaminoxid und Mischungen davon, wobei die Zusammensetzung vorzugsweise ein nichtionisches Tensid umfasst, das aus der Gruppe ausgewählt ist, bestehend aus Alkylpolyglucosid, alkoxyliertem Alkohol und Mischungen davon.

7. Antimikrobielle Zusammensetzung nach einem der vorstehenden Ansprüche, wobei das nichtionische Tensid ein Alkylpolyglucosid umfasst.

8. Antimikrobielle Zusammensetzung nach einem der Ansprüche 4 bis 7, wobei das nichtwässrige Lösungsmittel aus der Gruppe ausgewählt ist, bestehend aus Glycolethern, Diolen und Mischungen davon, vorzugsweise das nichtwässrige Lösungsmittel aus der Gruppe ausgewählt ist, bestehend aus Diethylenglycolbutylether, Di(propylenglycol)methylether und Mischungen davon.

9. Antimikrobielle Zusammensetzung nach einem der Ansprüche 4 bis 8, umfassend:

   a. zu etwa 0,01 bis etwa 10 Gew.-% der Zusammensetzung ein nichtionisches Tensid, das aus der Gruppe

ausgewählt ist, bestehend aus Alkylpolyglucosid, alkoxyliertem Alkohol und Mischungen davon; und
b. zu etwa 0,01 bis etwa 10 Gew.-% der Zusammensetzung ein nichtwässriges Lösungsmittel, das aus der Gruppe ausgewählt ist, bestehend aus Diethylenglycolbutylether, Di(propylenglycol)methylether und Mischungen davon.

**10.** Antimikrobielle Zusammensetzung nach einem der Ansprüche 4 bis 9, wobei die Zusammensetzung zu mindestens 80 Gew.-% der Zusammensetzung Wasser umfasst.

**11.** Antimikrobielle Zusammensetzung nach einem der Ansprüche 4 bis 10, wobei die Zusammensetzung in der Form einer klaren Flüssigkeit ist.

**12.** Antimikrobielle Zusammensetzung nach einem der Ansprüche 4 bis 11, wobei die Zusammensetzung in der Form eines gebrauchsfertigen Sprays ist.

**13.** Antimikrobielle Zusammensetzung nach einem der Ansprüche 4 bis 12, wobei die Zusammensetzung einen zusätzlichen Wirkstoff umfasst, der aus der Gruppe ausgewählt ist, bestehend aus einem Duftstoffrohstoff einschließlich Aldehyden und/oder Ketonen, einem zusätzlichen antimikrobiellen Wirkstoff, einem Pestizid, einem Insektenrepellent, einem Antimykotikum, einem antiviralen Mittel, einem herbiziden Mittel, einem Abtönungsfarbstoff, einem Antioxidationsmittel, einem nichtduftenden organoleptischen Stoff, einem Polymer, einem Schleifmittel, einem Stabilisierungsmittel, einem Bitterstoff, einer Mikrokapsel oder einer Kombination davon.

**14.** Artikel, der mit einer antimikrobiellen Zusammensetzung nach einem der Ansprüche 4 bis 12 behandelt ist, wobei der Artikel in der Form eines Einweg- oder teilweise wiederverwendbaren Substrats ist, umfassend eine oder mehrere Vliesschichten, und das Substrat einen Beladungsfaktor von etwa einem 3-fachen bis zu etwa einem 10-fachen der Zusammensetzung pro Gramm des Vliesstoffsubstrats aufweist.

**15.** Verfahren zum Desinfizieren einer unbelebten Oberfläche, umfassend den Schritt des Inkontaktbringens der Oberfläche mit einer antimikrobiellen Zusammensetzung oder einem Artikel nach einem der Ansprüche 4 bis 14.

**16.** Verwendung einer Zusammensetzung oder eines Artikels nach einem der Ansprüche 4 bis 14, um einer unbelebten Oberfläche antimikrobielle Vorteile bereitzustellen.

## Revendications

**1.** Ester de pyridinium selon la formule I

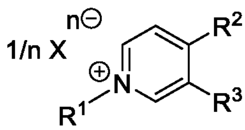

Formule I,

dans lequel 1/n $X^{n-}$ est un contre-ion et n vaut 1 ou 2 ;
dans lequel l'un parmi $R^2$ et $R^3$ est H et l'autre est choisi dans le groupe constitué par la Formule II, la Formule III et la Formule IV

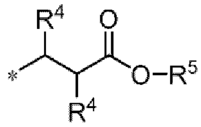

Formule II

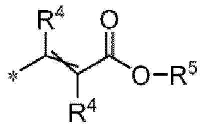

Formule III

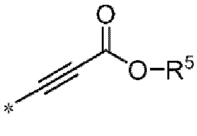

Formule IV

dans lequel * représente le point d'attachement au cycle hétéroaryle,
dans lequel la Formule III est choisie dans le groupe constitué par une configuration Z, une configuration E, et des mélanges de celles-ci, de préférence une configuration E,
dans lequel chaque $R^4$ est indépendamment choisi parmi hydrogène et un groupe hydrocarbyle en $C_1$ à $C_4$

et
dans lequel $R^5$ est un groupe hydrocarbyle en $C_4$ à $C_{12}$ ramifié ou non ramifié, saturé ou insaturé, de préférence $R^5$ est un groupe hydrocarbyle saturé en $C_6$ à $C_8$ ramifié ou non ramifié
dans lequel lorsque $R^2$ est H, $R^1$ est un groupe hydrocarbyle en $C_4$ à $C_{12}$ ramifié ou non ramifié, saturé ou insaturé ;
lorsque $R^3$ est H, $R^1$ est un groupe hydrocarbyle en $C_1$ à $C_{12}$ ramifié ou non ramifié, saturé ou insaturé.

**2.** Ester de pyridinium selon la revendication 1 dans lequel l'un parmi $R^2$ et $R^3$ est H et l'autre est la Formule III, de préférence la Formule III est

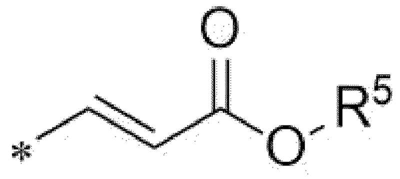

plus préférablement, $R^5$ est choisi parmi un groupe hydrocarbyle saturé en $C_6$ à $C_8$ ramifié ou non ramifié.

**3.** Ester de pyridinium selon l'une quelconque des revendications 1 ou 2 dans lequel l'ester de pyridinium est choisi dans le groupe constitué par :

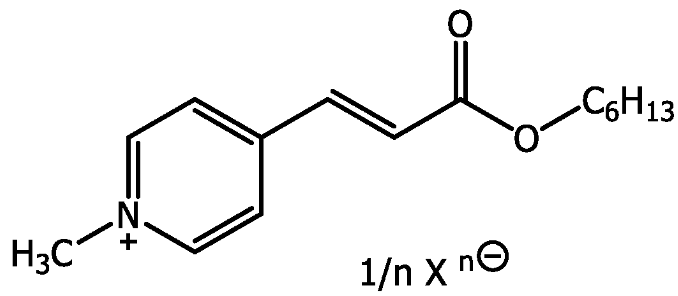

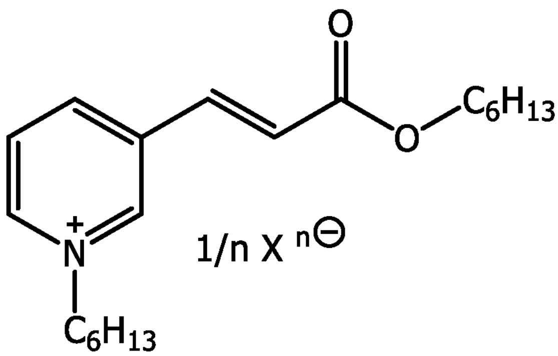

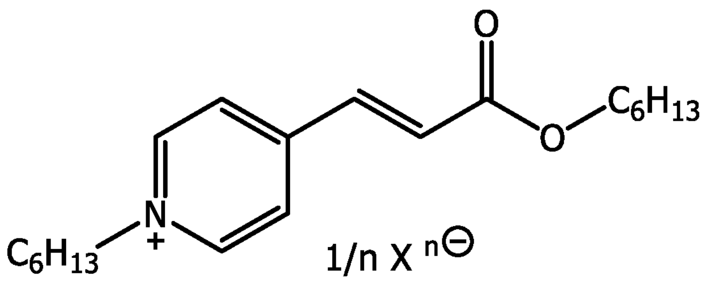

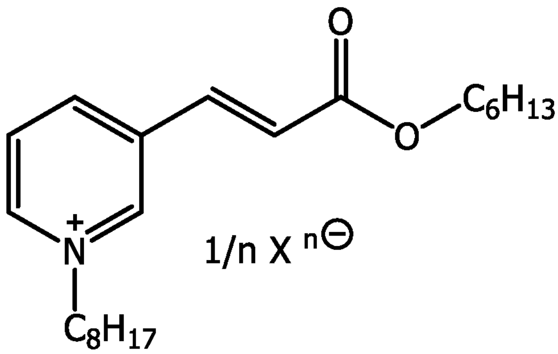

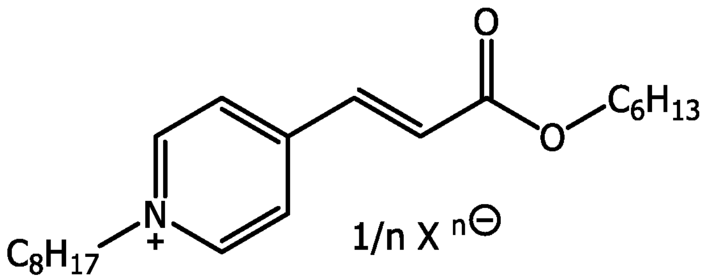

et un mélange de ceux-ci.

4. Composition antimicrobienne comprenant un système antimicrobien, le système antimicrobien comprenant :

a. un agent tensioactif ;
b. un solvant ; et
c. un ester de pyridinium selon l'une quelconque des revendications précédentes.

5. Composition antimicrobienne selon la revendication précédente dans laquelle le système antimicrobien comprend :

a. d'environ 0,01 à environ 10 % en poids de la composition d'un agent tensioactif choisi dans le groupe constitué par agent tensioactif non ionique, agent tensioactif cationique, agent tensioactif amphotère et un mélange de ceux-ci ;
b. de préférence d'environ 0,01 à environ 10 % en poids de la composition d'un solvant non aqueux ;
c. un ester de pyridinium selon l'une quelconque des revendications 1 à 3, de préférence un ester de pyridinium selon la revendication 3 ; et facultativement

au moins 50 % en poids de la composition d'eau ;

6. Composition antimicrobienne selon l'une quelconque des revendications 4 ou 5 dans laquelle la composition comprend un agent tensioactif non ionique ou un agent tensioactif amphotère choisi dans le groupe constitué par alkyl-polyglucoside, alcool alcoxylé, alkyl-bétaïne, alkyl-glucamine, alkyl-glucamide, oxyde d'alkyl-amine et mélanges de ceux-ci, de préférence la composition comprend un agent tensioactif non ionique choisi dans le groupe constitué par alkyl-polyglucoside, alcool alcoxylé et mélanges de ceux-ci.

7. Composition antimicrobienne selon la revendication précédente dans laquelle l'agent tensioactif non ionique comprend un alkyl-polyglucoside.

8. Composition antimicrobienne selon l'une quelconque des revendications 4 à 7 dans laquelle le solvant non aqueux est choisi dans le groupe constitué par éthers de glycol, diols et mélanges de ceux-ci, de préférence le solvant non aqueux est choisi dans le groupe constitué par éther butylique de diéthylène glycol, éther méthylique de di(propylène glycol) et mélanges de ceux-ci.

9. Composition antimicrobienne selon l'une quelconque des revendications 4 à 8 comprenant :

a. d'environ 0,01 à environ 10 % en poids de la composition d'un agent tensioactif non ionique choisi dans le groupe constitué par alkyl-polyglucoside, alcool alcoxylé et mélanges de ceux-ci ; et
b. d'environ 0,01 à environ 10 % en poids de la composition d'un solvant non aqueux choisi dans le groupe constitué par éther butylique de diéthylène glycol, éther méthylique de di(propylène glycol) et mélanges de ceux-ci.

**10.** Composition antimicrobienne selon l'une quelconque des revendications 4 à 9 dans laquelle la composition comprend au moins 80 % en poids de la composition d'eau.

**11.** Composition antimicrobienne selon l'une quelconque des revendications 4 à 10 dans laquelle la composition est sous la forme d'un liquide limpide.

**12.** Composition antimicrobienne selon l'une quelconque des revendications 4 à 11 dans laquelle la composition est sous la forme d'une pulvérisation prête à l'emploi.

**13.** Composition antimicrobienne selon l'une quelconque des revendications 4 à 12, dans laquelle la composition comprend un agent à effet bénéfique supplémentaire choisi dans le groupe constitué par une matière première de parfum comportant des aldéhydes et/ou des cétones, un agent antimicrobien supplémentaire, un pesticide, un répulsif pour insectes, un agent antifongique, un agent antiviral, un agent herbicide, une teinture teintante, un antioxydant, un organoleptique non-parfum, un polymère, un abrasif, un agent stabilisant, un agent d'amertume, une microcapsule ou une combinaison de ceux-ci.

**14.** Article traité avec une composition antimicrobienne selon l'une quelconque des revendications 4 à 12 dans lequel l'article est sous la forme d'un substrat jetable ou partiellement réutilisable comprenant une ou plusieurs couches de non-tissé et le substrat a un facteur de chargement allant d'environ 3 fois à environ 10 fois de composition par gramme de substrat non tissé.

**15.** Procédé d'assainissement d'une surface inanimée comprenant l'étape de mise en contact de la surface avec une composition antimicrobienne ou un article selon l'une quelconque des revendications 4 à 14.

**16.** Utilisation d'une composition ou d'un article selon l'une quelconque des revendications 4 à 14 pour fournir des effets bénéfiques antimicrobiens à une surface inanimée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021022286 A1
- WO 2002060438 A1
- WO 2001085714 A1
- EP 16184415 A
- US 20190055496 A
- US 2082275 A
- US 2702279 A
- US 2255082 A
- EP 2272942 A
- EP 2025743 A
- US 479096
- US 5549589 A
- US 5550167 A
- US 370695 A


### Non-patent literature cited in the description


- *Synlett*, 2011 (12), 1723-1726
- *Prepared as described in: Journal of the American Chemical Society*, 2011, vol. 133 (3), 582-594
- *Organic Letters*, 1999, vol. 1 (4), 579-582
- *Bioorganic & Medicinal Chemistry*, 2009, vol. 17 (5), 2047-2068
- *Journal of Heterocyclic Chemistry*, 1981, vol. 18 (3), 519-23
- *Zhurnal Organicheskoi Khimii*, 1976, vol. 12 (2), 443-8
- *Tetrahedron Letters*, 2018, vol. 59 (1), 14-17
- *Chemistry - A European Journal*, 2013, vol. 19 (45), 15281-15289
- *Chemistry - A European Journal*, 2009, vol. 15 (18), 4538-4542
- *ChemPlusChem*, 2016, vol. 81 (8), 893-898
- *ACS Catalysis*, 2022, vol. 12 (4), 2434-2440
- *Chemical Communications*, 2020, vol. 56 (7), 1101-1104
- Surfactant Science and Technology. Wiley Press
- **MCCUTCHEON'S**. *Detergents and Emulsifiers*, 1980
- *CHEMICAL ABSTRACTS*, # 3380-34-5
- *European Journal of Medicinal Chemistry*, 2011, vol. 46, 773-777